(19) Europäisches Patentamt
European Patent Office
Office européen des brevets

(11) **EP 3 825 657 A1**

(12) **EUROPÄISCHE PATENTANMELDUNG**

(43) Veröffentlichungstag:
26.05.2021 Patentblatt 2021/21

(51) Int Cl.:
*G01D 5/14* (2006.01)   *G01D 5/12* (2006.01)
*G01D 5/20* (2006.01)   *F16C 32/04* (2006.01)

(21) Anmeldenummer: 19211040.1

(22) Anmeldetag: 22.11.2019

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
**KH MA MD TN**

(71) Anmelder: **Berlin Heart GmbH**
**12247 Berlin (DE)**

(72) Erfinder: **PETERS, Oliver**
**14129 Berlin (DE)**

(74) Vertreter: **Pfenning, Meinig & Partner mbB**
**Patent- und Rechtsanwälte**
**Joachimsthaler Straße 10-12**
**10719 Berlin (DE)**

(54) **ROTATIONSMASCHINE MIT SENSORLOSER ROTORLAGEDETEKTION**

(57)      Rotationsmaschine mit einem Stator (13) und einem drehbar gelagerten Rotor (11), wobei in einem radialen Abstand von einer feststehenden Achse (20) ein oder mehrere Magnetfeldsensoren (12) bezüglich des Stators (13) feststehend angeordnet sind, mindestens einer Messvorrichtung (1), die ausgebildet ist, Magnetfeldänderungen mit Hilfe der vorgenannten Magnetfeldsensoren (12) zu erfassen, einem Rotor (11), der ausgebildet ist, mit einer oder mehreren konstanten magnetischen Quellspannungen und mit einem oder mehreren der Magnetfeldsensoren (12) jeweils ein oder mehrere elektrische Signale zu erzeugen, die Signalkomponenten aufweisen, die zur Rotordrehfrequenz (171) und zum jeweiligen Abstand zwischen Magnetfeldsensor (12) und Rotor (11) korrespondieren, gekennzeichnet durch eine Demodulatoreinheit (4), die ausgebildet ist, bei von den Magnetfeldsensoren (12) erzeugten oder daraus abgeleiteten Signalen eine Demodulation durchzuführen, so dass ein zum Abstand zwischen dem Rotor (11) und dem dem jeweiligen Signal zugeordneten Magnetfeldsensor (12) korrespondierendes Signal (140) erzeugt wird.

Figur 2

EP 3 825 657 A1

**Beschreibung**

Die Anmeldung betrifft eine Rotationsmaschine nach dem Anspruch 1. Ferner betrifft sie ein Verfahren zum Betrieb der Rotationsmaschine und ein System mit einer Rotationsmaschine.

Rotationsmaschinen zeichnen sich aus durch mindestens einen fest angeordneten Teil, der im Allgemeinen als Stator bezeichnet wird, und durch mindestens einen rotierenden Teil, der im Allgemeinen als Rotor bezeichnet wird. Beispiele für Rotationsmaschinen sind unter anderem Motoren, Kompressoren, Turbinen. Ihre Einsatzfelder sind vielfältig. Zum Beispiel können Motoren oder Turbinen als Antriebe eingesetzt werden, die wiederum andere Arbeit verrichtende Maschinen, wie beispielsweise Kompressoren oder Pumpen, antreiben. Rotationsmaschinen können dementsprechend selbst als Antrieb eingesetzt werden oder aber auch als angetriebene Maschine. Auch eine Kombination von Antriebs- und Arbeitsmaschine ist möglich, beispielsweise bei Motorkompressoren oder bei Pumpen, wie sie beispielsweise in Herzunterstützungssystemen eingesetzt werden und bei denen ein Motor und eine Pumpe in ein System integriert sind. Dabei ist es auch möglich, dass Pumpen- und Motor-Funktionalität mit nur einem Rotor realisiert werden.

Als Zwischenelement zwischen beweglichem Rotor und fest angeordnetem Stator dienen im allgemeinen Lager, wie beispielsweise Wälzlager, Gleitlager oder auch Magnetlager. Durch Kraftwirkungen auf den Rotor, wie sie beispielsweise bei Rotation durch Unwuchtkräfte oder während des Einsatzes durch externe Kräfte entstehen, können Rotoren schwingen. Die Schwingungen enthalten dabei häufig sowohl Komponenten, die zur Drehfrequenz des Rotors korrespondieren und neben der eigentlichen Drehfrequenz auch Harmonische der Drehfrequenz enthalten. Darüber hinaus besteht jedoch auch die Möglichkeit, dass Rotoren mit weiteren, nicht zur Drehfrequenz synchronen Frequenzen schwingen, beispielsweise mit Resonanzfrequenzen, deren Schwingfrequenz sowohl vom Rotor selbst als auch von der Lagerung des Rotors abhängt. Ob und welche dieser Resonanzfrequenzen angeregt werden, hängt unter anderem auch von den Krafteinwirkungen auf den Rotor ab.

Prinzipiell werden diese Schwingungen über die Lager auf den Stator übertragen, so dass diese Schwingungen als Vibrationen auch am Stator mit Vibrationssensoren messtechnisch erfasst werden können. Für den Dauerbetrieb werden für die zulässigen Schwingwerte im Allgemeinen Grenzwerte vorgegeben.

Im Allgemeinen ist man insofern daran interessiert, die Amplitude der Schwingungen sowohl im Rotor als auch im Stator gering zu halten, um Verschleiß und Funktionsbeeinträchtigungen entgegenzuwirken. Insbesondere bei Magnetlagermaschinen, d.h., Maschinen bei denen der Rotor durch Magnetkräfte in einer bezüglich des Stators definierten Sollposition gehalten werden soll, besteht häufig eine höhere Beweglichkeit des Rotors, die durch einen Spalt zwischen dem feststehenden Stator und dem drehbar gelagerten Rotor vorgegeben ist. Zur Erfassung des Schwingungsniveaus und unter Umständen auch zum aktiven Gegenwirken werden die Schwingungen des Rotors häufig permanent gemessen, insbesondere mit am Stator fest angeordneten Abstandssensoren, die den relativen Abstand zwischen Sensor und Rotor und damit auch zwischen Stator und Rotor messen, so dass aus dem zeitlichen Verlauf dieses Abstandes auf die Rotorschwingungen zurückgeschlossen werden kann.

Obwohl diese Methodik weit verbreitet ist, besteht ein wesentlicher Nachteil darin, dass zur Erfassung der Rotorposition eine vollständige Messkette notwendig ist und darüber hinaus Platz für die Sensorik im Maschinenaufbau bereitgestellt werden muss. Deshalb kann es in bestimmten Ausführungsformen vorteilhaft sein, die Rotorlageerfassung ohne zusätzliche Sensorik durchzuführen, d.h., die Rotorlage zumindest teilweise mit Mitteln durchzuführen, die ohnehin im Maschinenaufbau vorhanden sind. Man spricht in diesem Falle von sensorloser Rotorlagedetektion. Unter anderem sind solche Systeme in den Patentschriften US9506475B2 und US8226373B2 beschrieben.

Die Aufgabe der offenbarten Rotationsmaschine besteht darin, neben den Sensoren eine oder mehrere weitere oder alternativ andere Komponenten zur Rotorlagedetektion einzusparen. Diese Aufgabe wird durch eine Vorrichtung gemäß den Merkmalen des Anspruchs 1 und ein Verfahren gemäß den Merkmalen des Anspruchs 8 gelöst. Weitere vorteilhafte Ausführungsformen der Erfindung sind in den jeweiligen auf die unabhängigen Ansprüche zurück bezogenen Ansprüchen angegeben.

Die offenbarte Rotationsmaschine enthält neben einem Stator einen drehbar gelagerten Rotor, der ausgebildet ist, sich bezüglich des Stators zu bewegen. Für die Rotationsmaschine wird ferner eine bezüglich des Stators feststehende Achse definiert. In einem radialen Abstand dieser Achse sind ein oder mehrere Magnetfeldsensoren bezüglich des Stators feststehend angeordnet. Diese Magnetfeldsensoren können dabei explizit die spezielle Fähigkeit besitzen, Messungen für die Rotorlagedetektion durchzuführen. Sie können aber auch Teil anderer Komponenten sein, deren Haupteinsatzgebiet nicht die Durchführung von Messungen für die Rotorlagedetektion ist, sondern beispielsweise der Antrieb des Rotors. Die bezüglich des Stators feststehende Achse kann beispielsweise im Wesentlichen parallel zur Rotationsachse verlaufen und kann beispielsweise dazu genutzt werden, eine Rotorsollposition zu definieren.

Die Rotationsmaschine enthält darüber hinaus mindestens eine Messvorrichtung, die ausgebildet ist, Magnetfeldänderungen mit Hilfe der vorgenannten Magnetfeldsensoren zu erfassen. Die Messvorrichtung besitzt dabei auch die Eigenschaft, die von den Magnetfeldsensoren bereitgestellten Signale in Form von elektrischen Spannungen oder elektrischen Strömen so zu transformieren, dass sie für nachfolgende Verarbeitungsschritte geeignet sind. So kann die Messvorrichtung beispielsweise eine Impedanzanpassung beinhalten, eine Umsetzung von elektrischen Spannungen in elektrische Ströme oder von elektrischen Strömen in elektrische Spannungen, eine Reduzierung oder Anhebung der Amplituden von elektrischen Strömen oder Spannungen oder auch eine Analog-Digital-Umsetzung.

Ferner enthält die Rotationsmaschine einen Rotor, der ausgebildet ist, mit einer oder mehreren konstanten magnetischen Quellspannungen und mit einem oder mehreren der vorgenannten Magnetfeldsensoren jeweils ein oder mehrere elektrische Signale zu erzeugen, die Signalkomponenten aufweisen, die zur Rotordrehfrequenz und zum jeweiligen Abstand zwischen Magnetfeldsensor und Rotor korrespondieren. Dies bedeutet, dass auf dem Rotor magnetfelderzeugende Komponenten angeordnet sind, beispielsweise Permanentmagneten oder auch Elektromagneten. Bei Rotation erzeugen diese Magneten ein veränderliches magnetisches Feld. Die vorgenannten Magnetfeldsensoren sind so angeordnet, dass sie diesem veränderlichen Magnetfeld ausgesetzt sind und Signale erzeugen, die zur Stärke des zum jeweiligen Zeitpunkt vorhandenen Magnetfeldes oder dessen zeitlicher Änderung und räumlicher Ausrichtung korrespondieren. Diese Anordnung ist typisch für Synchronmaschinen aber auch für bürstenlose Gleichstrommotoren und generell für Maschinen, auf deren Rotor beispielsweise Magneten, beispielsweise Permanentmagneten, angeordnet sind. Die Magnetfeldsensoren können in diesem Falle beispielsweise durch Motorspulen gebildet werden, die im Allgemeinen vom Magnetfeld der auf dem Rotor angeordneten Magneten durchdrungen werden. Insbesondere bei Rotation des Rotors ergibt sich ein wechselndes Magnetfeld, was zu einer Induktion von elektrischer Spannung in den Motorspulen führt.

Die aus dem mit Magneten ausgerüsteten Rotor und den Magnetfeldsensoren bestehende Anordnung kann auch als Amplituden-Modulator interpretiert werden. Das durch die Rotordrehung erzeugte magnetische Wechselfeld erzeugt in den Magnetfeldsensoren, die beispielsweise als Motorspulen ausgeführt sind, eine elektrische Wechselspannung, die im Falle von Spulen proportional zur Änderung des magnetischen Flusses in den Spulen ist. Der magnetische Fluss ist dabei zum einen abhängig von der aktuellen Position des Magneten in Bezug auf die jeweilige Spule. Zum anderen ist der magnetische Fluss jedoch auch abhängig von der Konfiguration des zugehörigen magnetischen Kreises, insbesondere von den verwendeten Materialien sowie den im magnetischen Kreis existierenden Spalten, die Materialien enthalten, die nicht oder nur gering magnetisch leitfähig sind. Insbesondere ist der magnetische Fluss vom Abstand zwischen der jeweiligen Spule und dem jeweiligen Rotormagneten abhängig. Deshalb wird der magnetische Fluss durch die Rotorposition bezüglich einer Spule und dementsprechend auch die elektrische Spannung in der jeweiligen Spule durch den Abstand zwischen Rotor und Spule moduliert. Das entstehende Spannungssignal weist deshalb die Merkmale eines amplitudenmodulierten Signals auf, das durch eine Trägerschwingung und ein Modulationssignal, welches die Trägerschwingung moduliert, charakterisiert wird. Die Frequenz der Trägerschwingung, die Trägerschwingungsfrequenz, ist in diesem Fall die Frequenz, die sich aus dem Produkt von Rotordrehfrequenz und Polpaarzahl des Rotors ergibt und das Modulationssignal ist der jeweilige Abstand zwischen Spule und Rotor. Die Trägerschwingungsfrequenz liegt damit in einem Bereich, der mit dem Drehzahlbereich des Motors überlappt oder eng benachbart zum Drehzahlbereich des Motors ist. Die Polpaarzahl des Rotors bezieht sich auf die magnetischen Pole des Rotors, die in der Nähe des jeweiligen Magnetfeldsensor angeordnet sind und deren Magnetfeld bei Rotation ausgebildet ist, in den Magnetfeldsensoren eine Spannung zu induzieren. Die Polpaarzahl errechnet sich aus der Anzahl dieser magnetischen Pole geteilt durch zwei.

Der Rotor mit der beschriebenen Anordnung ist dazu ausgebildet, mit seinen Magneten und dem Abstand zum Magnetfeldsensor ein abstandsmoduliertes Signal erzeugen zu können. Dies ist insbesondere interessant bei Motoren, bei denen Magneten ohnehin auf dem Rotor platziert sind, wie beispielsweise Synchronmotoren oder bürstenlose Gleichstrommotoren.

Die Rotationsmaschine enthält im Weiteren eine Demodulatoreinheit, die ausgebildet ist, bei von den Magnetfeldsensoren erzeugten oder daraus abgeleiteten Signalen, die Signalkomponenten aufweisen, die zur Rotordrehfrequenz und zum jeweiligen Abstand zwischen Magnetfeldsensor und Rotor korrespondieren, eine Demodulation durchzuführen, so dass ein zum Abstand zwischen dem Rotor und dem dem jeweiligen Signal zugeordneten Magnetfeldsensor korrespondierendes Signal erzeugt wird

Bei der Demodulatoreinheit handelt es sich um eine Komponente, die beispielsweise eine Amplitudendemodulation durchführt. Die Amplitudendemodulation eines Signals umfasst im Allgemeinen die Umsetzung eines Signalfrequenzbandes, das um eine Trägerschwingungsfrequenz definiert ist, in den Bereich um die Frequenz 0 Hz. Die praktische Umsetzung kann beispielsweise mit einem bekannten Einhüllendendemodulator erfolgen oder aber auch durch Multiplikation mit einem sinusförmigen Signal, das die Frequenz der Trägerschwingung aufweist, und anschließende Tiefpassfilterung. Darüber hinaus sind weitere Demodulationsverfahren bekannt, beispielsweise, in dem statt mit einem sinusförmigen Signal mit einem periodischen Signal multipliziert wird, wobei die Periodenfrequenz oder eine Harmonische der Trägerschwingungsfrequenz entspricht. Die beschriebene Multiplikation kann beispielsweise mit einer elektronischen Mischerschaltung durchgeführt werden. Darüber hinaus kann die Umsetzung eines Frequenzbandes mit Hilfe der Fouriertransformation ausgeführt werden. Ein solches Verfahren wird bevorzugt im digitalen, d.h. zeit- und amplitudendiskreten Bereich angewendet, da hier für die Berechnung der Fouriertransformation schnelle und effiziente Algorithmen (Fast Fourier Transform - FFT) verfügbar sind.

Es besteht ferner die Möglichkeit, dass bei der Rotationsmaschine die Magnetfeldsensoren als Spulen ausgeführt und/oder als Motorspulen ausgeführt sind und ausgebildet sind, Magnetfeldänderungen zu erfassen und ein für den Antrieb des Rotors geeignetes Magnetfeld zu erzeugen. Werden Sensorspulen als Magnetfeldsensoren genutzt, ist die jeweils induzierte und an den Spulenklemmen messbare Spannung proportional zur Änderung des magnetischen Flusses in der Spule. Werden stromführende Motorspulen oder Lagerspulen als Magnetfeldsensoren genutzt, ist die an den Spulenklemmen messbare Spannung die Summe aus der Induktionsspannung durch die Änderung des magnetischen Flusses in der Spule, der Selbstinduktionsspannung, hervorgerufen durch die Induktivität der Spule und dem resistiven Spannungsabfall am Widerstand des Spulendrahtes. Eine größere Amplitude des magnetischen Wechselflusses führt somit zu einer proportional größeren Wechselspannungskomponente an den Klemmen der Spule. Die Magnetfeldsensoren können auch als differentielle Hallsensoren ausgeführt sein. Die von den Hallsensoren generierten Signale sind proportional zur magnetischen Fluss.

In einem Elektromotor können die Spulen, die ein für den Antrieb des Rotors geeignetes Magnetfeld erzeugen, d.h. die Motorspulen, gleichzeitig für die Erfassung von Magnetfeldänderungen genutzt werden. In diesem Fall können beispielsweise bereits im Motor vorhandene Komponenten diese zusätzliche sensorische Aufgabe übernehmen, gesonderte Sensoren werden also nicht benötigt. Alternativ können aber trotzdem auch andere Sensoren als Magnetfeldsensoren Verwendung finden, beispielsweise differentielle Hallsensoren oder Spulen, die keine primär dem Antrieb zugeordnete Motorspulen sind.

Die Rotationsmaschine kann auch mit einer Vorrichtung ausgerüstet sein, die die Rotordrehfrequenz zur Verfügung stellt. Dabei besteht die Möglichkeit, dass die Rotordrehfrequenz von der Motorsteuerung zur Verfügung gestellt wird, da diese Information dort gegebenenfalls ohnehin zur Verfügung steht. Darüber hinaus ist aber auch eine Messung der Rotordrehfrequenz möglich, beispielsweise indem eine oder mehrere Markierungen auf dem Rotor aufgebracht und im Betrieb von geeigneten Sensoren erfasst werden. Ein mögliches Beispiel für eine Vorrichtung zur Messung der Rotordrehfrequenz ist unter anderem eine schmale Nut als Markierung und ein auf den Rotor ausgerichteter Abstandssensor, ein sogenannter Keyphasor, der so eingestellt ist, dass er bei Passieren der Nut, die auch als Keyphasor-Nut bezeichnet wird, ein Spannungssignal ausgibt, dass sich von dem von ihm ausgegebenen Spannungssignal in den Phasen des Rotorumlaufs unterscheidet, in denen sich Keyphasor und Keyphasor-Nut nicht gegenüberstehen. Mit einer Vergleichsvorrichtung kann das vom Keyphasor erzeugte Spannungssignal in einen kurzen Spannungsimpuls pro Rotorumlauf umgesetzt werden, der anschließend von einer Verarbeitungseinheit, beispielsweise einer Zählerbaugruppe, in ein Drehzahlsignal umgesetzt werden kann. Ein ähnliches Verfahren ist beispielsweise auch auf optischer Basis möglich. Das Drehzahlsignal kann letztlich in verschiedener Form vorliegen, beispielsweise als Spannung, Strom, als Zahlenwert, in Form eines Impulses pro Umlauf oder auch als periodisches Signal mit einer zur Rotordrehfrequenz korrespondierenden Frequenz. Prinzipiell kann die Rotordrehfrequenz auch mit einer Frequenzanalyse, beispielsweise einer Fouriertransformation durchgeführt werden. Dabei wird ein Magnetsensorsignal in den Frequenzbereich transformiert und mittels einer Peakerkennung die Drehfrequenz detektiert.

Optional ist die Demodulatoreinheit ausgebildet, die Rotordrehfrequenz bei der Demodulation zu verwenden. Dies ist insbesondere dann der Fall, wenn, im Unterschied zur Einhüllendendemodulation, die Demodulation im digitalen Bereich mittels eines Computers oder Mikrocontroller unter Verwendung der Fouriertransformation, insbesondere der Diskreten Fouriertransformation mit ihrer effizienten Implementierung, der schnellen Fouriertransformation oder auch beispielsweise mit dem Goertzel-Algorithmus, durchgeführt wird.

Dazu wird das zu demodulierende Signal mit Hilfe der Fouriertransformation in den Frequenzbereich transformiert. Bei der Diskreten Fouriertransformation liegt die Fouriertransformierte des Signals in einem abgetasteten Zustand, d.h. in Form diskreter Werter an Frequenzstützstellen vor. Für die Demodulation werden zunächst alle Werte an den Frequenzstützstellen, die nicht zur Trägerschwingungsfrequenz, die zur Rotordrehfrequenz korrespondiert, und dem amplitudenmodulierten Signal gehören, maskiert, d.h. zu Null gesetzt. Die verbliebenen, nicht maskierten Frequenzstützstellen, werden um den Betrag der Trägerschwingungsfrequenz zur Frequenz 0 Hz hin verschoben. Alle zum Bereich um die Frequenz 0 Hz verschobenen Signalanteile werden aufaddiert und das resultierende Signal in den Zeitbereich zurücktransformiert. Der Vorteil dieser Vorgehensweise liegt in seiner Einfachheit. Der Nachteil besteht darin, dass die Phasenlage des Abstandssignals verloren geht und somit eine Information, die für die Generierung von Steuersignalen in bestimmten Ausführungsformen wichtig sein kann. Es besteht noch die Möglichkeit, zumindest die Leistung der Rotorschwingungen zu bestimmen. Da die Leistung prinzipiell gemäß dem Parseval-Theorem auch im Frequenzbereich bestimmt werden kann, kann die Leistung der Rotorschwingung jedoch auch direkt im Frequenzbereich bestimmt werden. Die Leistung der Schwingung kann genutzt werden, um das jeweils aktuelle Schwingungsniveau zu bewerten und darauf basierende Steuersignale, wie beispielsweise eine Notabschaltung, zu erzeugen.

Die Rotationsmaschine kann auch mit einer Vorrichtung ausgerüstet sein, die den Rotordrehwinkel zur Verfügung stellt. Der Rotordrehwinkel ist ein durch Rotordrehung entstehender Winkel, der in einer Ebene senkrecht zur Rotationsachse des Rotors, der Winkelebene, definiert ist und der sich aus der aktuellen Position eines Referenzpunktes auf dem Rotor, einer Referenzposition dieses Referenzpunktes in Bezug auf den Stator und dem Durchtrittspunkt der Rotationsachse des Rotors durch die Winkelebene, der Scheitelpunkt des Rotordrehwinkels ist, ergibt. Als Referenzpunkt kann beispielsweise die Keyphasor-Nut verwendet werden, die auch zur Drehzahlbestimmung genutzt wird. Als Referenzposition bezüglich des Stators ist beispielsweise die Position des Keyphasors geeignet. Der aktuelle Rotordrehwinkel wird schließlich mit Hilfe der aktuellen Drehzahl bestimmt, mit

$$\text{Rotordrehwinkel} = (\text{Rotordrehfrequenz} * dt * 360 \bmod 360)°,$$

wobei dt die verstrichene Zeit ist, seit dem der Referenzpunkt des Rotors letztmalig die Referenzposition bezüglich des Stators passiert hat. Die Zeit kann beispielsweise mit einer Uhr oder einer Zählerbaugruppe gemessen werden.

Mit bekanntem Rotordrehwinkel kann das vorherig beschriebene Verfahren zur Demodulation im Frequenzbereich um eine Phasenkorrektur ergänzt werden. Phasenkorrektur bedeutet in diesem Fall, dass auf den Phasenwinkel jedes Fourierkoeffizienten ein vom Rotordrehwinkel abhängiger Offset addiert wird, so dass bei der Rücktransformation in den Zeitbereich die Phasenlage des Signals der Phasenlage der Rotorschwingungssignals entspricht.

Darüber hinaus ist es mit dem Rotordrehwinkel möglich, die Demodulation des Signals durch Multiplikation mit einer sinusförmigen Schwingung mit Hilfe einer elektronischen Schaltung durchzuführen. Dazu wird beispielsweise mit Hilfe eines Oszillators eine Frequenz erzeugt, die der Trägerschwingungsfrequenz entspricht und die die Phasenlage der erzeugten Frequenz, beispielsweise mit einem Phasenregelkreis, so eingestellt, dass sie der Phasenlage der Trägerschwingung entspricht. Die Phasenlage kann dabei direkt aus der Phasenlage der Trägerschwingung extrahiert werden oder kann alternativ auch aus dem Rotordrehwinkel berechnet werden.

Optional oder zusätzlich enthält die Rotationsmaschine eine erste Verarbeitungseinheit, die ausgebildet ist, ein oder mehrere elektrische Signale der vorgenannten Magnetfeldsensoren zu einem oder mehreren Signalen so zu überlagern und/oder zu filtern, dass der Signalanteil im jeweils resultierenden Signal, der eine Information zum Abstand zwischen dem Rotor und dem jeweiligen Magnetfeldsensor enthält, jeweils im Verhältnis zu anderen Signalkomponenten verstärkt wird.

Diese erste Verarbeitungseinheit kann als analoge Schaltung ausgeführt werden, und kann beispielsweise Addier- oder Subtrahierschaltungen oder auch Filterschaltungen enthalten, die beispielweise mit Operationsverstärkern realisiert sein können. Prinzipiell kann diese erste Verarbeitungseinheit aber auch teilweise oder vollständig digital ausgeführt sein, beispielsweise als digitale Recheneinheit auf der Basis von einem oder mehreren Mikrocontrollern, Prozessoren, Anwenderspezifischen Schaltungen oder auch in Field Programmable Gate Arrays oder alternativ auch mit diskreten Bauelementen.

Bei einer in der ersten Verarbeitungseinheit möglichen Signalüberlagerung der von den Magnetfeldsensoren gemessenen Signale besteht die Option, beispielsweise mehrere der gemessenen Signale phasenrichtig so aufzuaddieren, dass Signalkomponenten, die keine Information zur Lage des Rotors enthalten, ausgelöscht werden. Diese Möglichkeit kann in bestimmten Ausführungsformen vorteilhaft verwendet werden, wenn die Rotationsmaschine beispielsweise ein Elektromotor ist und antriebsbedingte Magnetfeldanteile einen hohen Anteil der gemessenen Magnetfeldleistung ausmachen oder rotorachslageunabgängige Signalkomponenten das Rotorlagesignal überlagern.

Bei einer in der ersten Verarbeitungseinheit möglichen Signalfilterung der von den Magnetfeldsensoren gemessenen Signale besteht die Option, beispielsweise frequenzselektive Filter, beispielsweise Tiefpässe, in bestimmten Ausführungsformen vorteilhaft einzusetzen, so dass alle Signalanteile, die außerhalb des Frequenzbandes liegen, das eine Information zur Rotorposition enthält, in den gemessenen Signalen unterdrückt, d.h. gedämpft werden. Solche zu unterdrückenden Signalanteile können beispielsweise Signalanteile sein, die aus der Motorsteuerung stammen. Motorsteuersignale können beispielsweise pulsweitenmoduliert sein, wobei die Schaltfrequenz der Pulsweitenmodulation beispielsweise mehrere tausend Hertz betragen kann. Durch Tiefpassfilterung können die Schaltfrequenz und auch deren Oberwellen unterdrückt werden.

Die Rotationsmaschine kann darüber hinaus eine zweite Verarbeitungseinheit enthalten, die dem Demodulator nachgeschaltet ist und die ausgebildet ist, aus den demodulierten Signalen ein oder mehrere Rotorpositionssignale zu erzeugen. Die demodulierten Signale enthalten ein zum Abstand zwischen dem Rotor und dem jeweiligen Magnetfeldsensor korrespondierendes Signal. Da die Änderung des magnetischen Flusses proportional zur Wechselfrequenz des Flusses ist, korrespondiert die Amplitude dieses Abstandssignals nicht nur zum Abstand zwischen Magnetfeldsensor und Rotor sondern auch zur Frequenz der Trägerschwingung, die wiederum mit der Rotordrehfrequenz in Zusammenhang steht. Um diese Abhängigkeit aus dem Signal zu eliminieren, wird zunächst eine drehzahlabhängige Skalierung des Abstandssignals durchgeführt und anschließend, mit Hilfe der bekannten Position der Magnetfeldsensoren, und den ermittelten Abstandsinformationen zwischen den Magnetfeldsensoren und Rotor die Rotorposition in Bezug auf ein statorfestes Koordinatensystem bestimmt. Dabei handelt es sich in der Regel, jedoch nicht notwendigerweise, um ein kartesisches Koordinatensystem. Auf Grundlage dieses Koordinatensystems werden Rotorpositionssignale erzeugt, die für jeden Messzeitpunkt die Koordinaten des Rotors in Bezug auf dieses Koordinatensystem darstellen. Vorzugsweise handelt es sich um Koordinaten, die die Rotorposition senkrecht zur Drehachse oder auch parallel zur Drehachse beschreiben.

Die Rotationsmaschine kann auch vorzugsweise eine Steuereinheit enthalten, die ausgebildet ist, aus den Rotorpositionssignalen Steuersignale zu generieren. Bei der Steuereinheit kann es sich beispielsweise um eine Überwachungseinheit handeln, die beispielsweise bei zu großen Rotorschwingungen eine Notabschaltung des Gesamtsystems vornimmt, eine Drehzahländerung auslöst, Steuerventile betätigt oder die Ausgabe von Alarmsignalen, beispielsweise optisch oder akustisch, veranlasst. Darüber hinaus kann eine Protokollierung der Rotorpositionssignale durchgeführt werden. Alternativ kann die Steuereinheit auch als Regeleinrichtung ausgebildet sein, die Steuersignale generiert, die über einen oder mehrere Aktuatoren eine oder mehrere Kräfte auf den Rotor ausübt, die die Rotorschwingungen, insbesondere die Lage oder die Geschwindigkeit des Rotors relativ zu den feststehenden Magnetfeldsensoren, beeinflussen. Bei den Aktuatoren kann es sich beispielsweise um ein Rüttelelement handeln, dass beispielsweise Schwingungen auf den Stator überträgt, wodurch beispielsweise über die Lagerung eine Kraft auf den Rotor übertragen werden kann, die zu einer Dämpfung der Rotorschwingungen führen kann. Voraussetzung für eine Dämpfungswirkung ist, dass die Vibration des Rüttelelements phasenrichtig erzeugt wird. Andere Aktuatoren zur Ausübung einer Kraft auf den Rotor sind beispielsweise Elektromagneten oder auch Piezo-Aktuatoren.

Für ein Monitoring, eine spätere Auswertung des Signale oder auch eine eventuelle Fehlersuche ist es sinnvoll, die Rotationsmaschine optional mit einer Datensammeleinheit auszurüsten oder zu verbinden, die ausgebildet ist, einen oder mehrere ermittelte Positionswerte des Rotors zu speichern. Die Datensammeleinheit kann dazu in oder an der Rotationsmaschine angebracht sein. Sie kann alternativ aber auch von der Rotationsmaschine räumlich getrennt angeordnet sein, beispielsweise auf einem entfernten Server.

Die Anmeldung für die Rotationsmaschine betrifft auch ein Verfahren, dass die vorherig beschriebene Anordnung verwendet. Wesentliche Kernpunkte des Verfahrens sind, dass in einem ersten Schritt an den Magnetfeldsensoren elektrische Signale gemessen werden und in einem zweiten Schritt diese oder daraus abgeleitete Signale demoduliert werden. Bei den elektrischen Signalen kann es sich um Ströme oder Spannungen handeln, die von den Magnetfeldsensoren erzeugt werden. Die Demodulation kann mit verschiedenen Verfahren durchgeführt werden, beispielsweise als Einhüllendendemodulation oder aber auch unter Nutzung der Rotordrehfrequenz im Zeit- oder Frequenzbereich, optional auch unter Nutzung des Rotordrehwinkels.

Vor der Demodulation ist ein optionaler Zwischenschritt vorsehbar, in dem ein oder mehrere elektrische Signale der vorgenannten Magnetfeldsensoren zu einem oder mehreren Signalen so verarbeitet werden, dass der Signalanteil im jeweils resultierenden Signal, der eine Information zum Abstand zwischen dem Rotor und dem jeweiligen Magnetfeldsensor enthält, jeweils im Verhältnis zu anderen Signalkomponenten verstärkt wird. Ausgeführt werden kann diese Aufgabe beispielsweise durch Anwendung frequenzselektiver Filter, wie beispielsweise Tiefpässe, oder auch durch Linearkombination einer oder mehrerer Signale der Magnetfeldsensoren.

Ferner besteht in einem weiteren Verfahrensschritt die Möglichkeit, dass aus den demodulierten Signalen eine Rotorposition und/oder eine lineare Verschiebungsgeschwindigkeit und/oder eine lineare Beschleunigung der Rotorachse ermittelt wird. Für die Bestimmung der Rotorposition wird vorzugsweise, ausgehend von den bekannten Positionen der Magnetfeldsensoren und mit Hilfe der ermittelten Abstände des Rotors zu den Magnetfeldsensoren, die Position des Rotors in Form von Koordinaten eines Koordinatensystems bestimmt.

In einem optionalen Verfahrensschritt können aus den Rotorpositionssignalen Steuersignale generiert werden. Diese Steuersignale können für eine Überwachung verwendet werden, um beispielsweise bei zu großen Schwingungswerten eine Notabschaltung herbeiführen zu können oder aber bestimmte Betriebsparameter zu ändern. Beispielsweise kann die Steuereinrichtung eine Drehzahländerung auslösen, Steuerventile ansteuern oder die Ausgabe von Alarmsignalen, beispielsweise optisch oder akustisch, bewirken. Darüber hinaus kann eine Protokollierung der Rotorpositionssignale ausgelöst werden.

Ferner können die Steuersignale auch dafür verwendet werden, um, beispielsweise mit Hilfe elektromagnetischer Aktuatoren, Piezo-Aktuatoren oder Rüttelelementen, die Lage oder die Geschwindigkeit des Rotors relativ zu den feststehenden Magnetfeldsensoren zu beeinflussen und so aktiv Schwingungen entgegenzusteuern. Aktuatoren können beispielsweise Elektromagneten in Form von Motorspulen sein oder auch die Elektromagneten eines aktiven Magnetlagers. Die Steuersignalerzeugung kann beispielsweise mit Hilfe eines Reglers erfolgen, der beispielsweise eine PID-Charakteristik aufweist oder auch optional um weitere Filterelemente ergänzt werden kann. Der Regler kann auch, optional, als multivariater Regler in einer Zustandsraum-Darstellung ausgeführt sein, in dem die Reglerparameter über ein Optimierungsverfahren, beispielsweise ein H∞-Verfahren bestimmt werden. Die Beeinflussung von Lage und Geschwindigkeit zielt darauf ab, zum einen die mittlere Position des Rotors zu beeinflussen als auch die Schwingungsneigung des Rotors zu dämpfen.

Wenn die Magnetfeldsensoren als Motorspulen mit einem Mittelabgriff ausgeführt sind, ist das offenbarte Verfahren optional ausgebildet, ein oder mehrere Steuersignale zur symmetrischen oder asymmetrischen Ansteuerung von einem oder mehreren Motorspulen zu erzeugen und auf diesem Wege die Lage oder die Geschwindigkeit des Rotors relativ zu den feststehenden Magnetfeldsensoren zu beeinflussen. Bei einer symmetrischen Ansteuerung wird der Steuerstrom so in die Motorspulen einer Phase eingeprägt, sodass die Ansteuerung an der Phasenklemme und damit für die Motorsteuerung nicht bemerkbar ist. Bei einer asymmetrischen Ansteuerung, bei beispielsweiser Ansteuerung von nur einer Motorspule eines Phasenzweiges, heben sich die durch die Ansteuerung bedingten Ströme an der Phasenklemme nicht auf.

Optional ist das offenbarte Verfahren ausgebildet, dass aus den Rotorpositionssignalen eine einwirkende Kraft oder ein einwirkendes Drehmoment auf den Rotor ermittelt wird. Die Rotorposition gibt nicht nur die Lage bezüglich eines statorfesten Koordinatensystems an, sondern auch die Lage bezüglich eines Lagers, dass zur Lagerung des Rotors verwendet wird. Beispielsweise können Wälz, Gleit- oder auch Magnetlager verwendet werden. Diese Lager weisen jeweils eine bekannte Steifigkeit auf, so dass, über den Proportionalitätsfaktor Steifigkeit, direkt eine Kraft bestimmt werden kann, mit der der Rotor in das Lager gedrückt wird. Darüber hinaus ist es möglich, mit dieser einwirkenden Kraft ein Drehmoment bezüglich eines rotorfesten Punktes zu bestimmen. Bei Verwendung von Aktuatoren für die Regelung der Rotorlage, muss die von den Aktuatoren ausgeübte Kraft bei der Bestimmung der auf den Rotor einwirkenden Kraft bzw. des Drehmoments berücksichtigt werden.

Optional kann das offenbarte Verfahren ausgebildet sein, dass anhand von Tabellen, welche im Pumpencontroller abgelegt sind, oder simpler, mehrdimensionaler Approximationen mit Polynomen n-ten Grades (vorzugsweise nicht höher als 4) aus der Rotorposition und dem auf den Rotor einwirkenden Drehmoment auf Strömungskenngrößen wie Druckverteilung, Druckerzeugung oder Flussmenge geschlossen wird. Dies kann unter anderem auch in Kombination mit der Drehzahl und Stromaufnahme des Motors und einer geschätzten Viskosität erfolgen. Ferner kann die Bewegung des Rotors insbesondere dessen Bewegungsfrequenzen dahingehend genutzt werden, um im Bereich der Pumpen Thromben zu detektieren und die Viskosität des Blutes zu schätzen.

Weiterhin kann die optional auf den Rotor einwirkende Kraft oder das auf den Rotor einwirkende Drehmoment ausgewertet werden, um auf Systemparameter wie Alterung, Verschleiß, Korrosion oder biologischen Bewuchs zu schließen.

Darüber hinaus können im Rahmen des offenbarten Verfahrens optional ein oder mehrere ermittelte Rotorpositionswerte in einer Datensammeleinheit gespeichert werden. Ferner ist es ebenfalls möglich, weitere Sekundärdaten wie die ermittelte Kraft, das Drehmoment sowie weitere geschätzte Parameter bezüglich Alterung, Verschleiß, Korrosion oder biologischen Bewuchs in der Datensammeleinheit zu speichern.

Im Folgenden werden anhand von Figuren Ausführungsbeispiele gezeigt und nachfolgend erläutert. Dabei zeigt

Figur 1: Systemüberblick;

Figur 2: Beispiel für einen Rotor und Magnetfeldsensoren in radialer Richtung;

Figur 3a: Beispiel für einen Rotor und Magnetfeldsensoren in axialer Richtung;

Figur 3b: Definition einer bezüglich des Stators feststehenden Achse;

Figur 4a: Beispiel für als Motorspulen ausgeführte Magnetfeldsensoren mit einem zweipoligen Rotor;

Figur 4b: Beispiel für als Motorspulen ausgeführte Magnetfeldsensoren mit einem vierpoligen Rotor;

Figur 4c: Beispiel für die Definition eines statorfesten Koordinatensystems;

Figur 5: Beispiel für die Verschaltung von Motorspulen und deren Nutzung für die Messung von Magnetfeldern;

Figur 6a: Beispiel für die Vorverarbeitung der Magnetfeldsensorsignale mit frequenzselektiver Filterung;

Figur 6b: Beispiel für die Vorverarbeitung der Magnetfeldsensorsignale mit frequenzselektiver Filterung und Signalkombination;

Figur 6c: Beispiel für die Vorverarbeitung der Magnetfeldsensorsignale mit Signalkombination;

Figur 7a: Beispiel für die Bestimmung der Rotordrehfrequenz und des Rotordrehwinkels mit Hilfe einer Zählerbaugruppe;

Figur 7b: Beispiel für die Bestimmung der Rotordrehfrequenz und des Rotordrehwinkels mit Hilfe der Motorsteuerung;

Figur 8a: Signalbeispiel für eine Amplitudenmodulation im Zeitbereich;

Figur 8b: Signalbeispiel für eine Amplitudenmodulation im Frequenzbereich;

Figur 9a: Signalbeispiel für eine Amplitudendemodulation im Zeitbereich;

Figur 9b: Signalbeispiel für eine Amplitudendemodulation im Frequenzbereich;

Figur 10a: Beispiel für Maskierung im Frequenzbereich;

Figur 10b: Beispiel für Frequenzverschiebung im Frequenzbereich;

Figur 10c: Beispiel für demoduliertes Signal im Frequenzbereich;

Figur 11: Beispiel für Trägersynthese durch Maskierung im Frequenzbereich;

Figur 12a: Beispiel für die Trägersynthese zur Amplitudendemodulation mit Hilfe einer Oszillatorschaltung;

Figur 12b: Beispiel für die Trägersynthese zur Amplitudendemodulation mit Hilfe der Fouriertransformation;

Figur 13a: Beispiel für die Demodulation mit Rotordrehfrequenz im Frequenzbereich ohne Rotordrehwinkel;

Figur 13b: Beispiel für die Demodulation mit Rotordrehfrequenz im Frequenzbereich mit Rotordrehwinkel;

Figur 13c: Beispiel für die Demodulation mit Rotordrehfrequenz und Rotordrehwinkel im Zeitbereich;

Figur 14: die Abbildung der demodulierten Magnetfeldsensorsignale auf ein Koordinatensystem und

Figur 15: Beispiel für die Nutzung der Rotorposition in einem geregelten Rotorlagesystem.

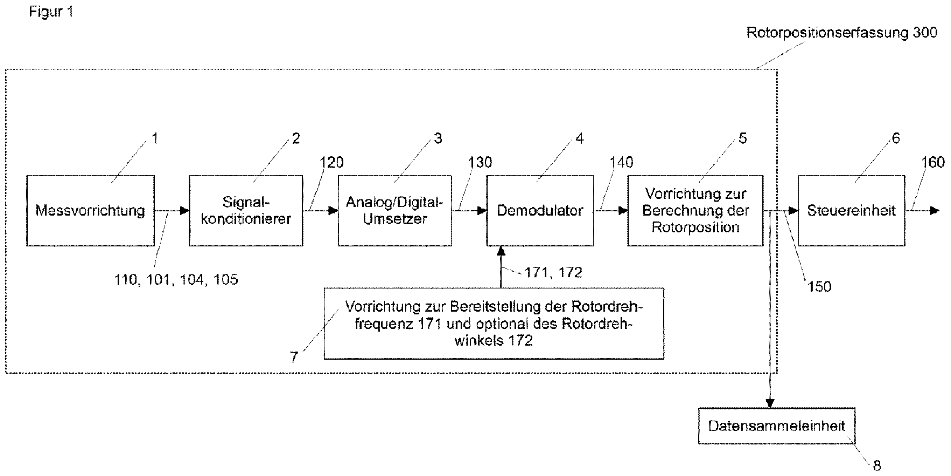

In Figur 1 werden grundlegenden Systemkomponenten der Rotorpositionserfassung 300, die Steuereinheit 6 und die Datensammeleinheit 8 dargestellt. Die Messvorrichtung 1 ist vor allem ausgebildet, auch in Überlagerung zu Motorsignalen, ein oder mehrere Magnetfelder oder Änderungen dieser Magnetfelder zu erfassen, in elektrische Strom- oder Spannungssignale umzuformen und diese Spannungssignale in eine Form zu bringen, dass sie weiter verarbeitet werden können. Die Messvorrichtung 1 umfasst hierzu Magnetfeldsensoren 12 sowie beispielsweise elektrische Verbinder und Anschlüsse und optional Messverstärker, Pegelumsetzer oder Impedanzwandler. Am Ausgang der Messvorrichtung 1 werden für den optionalen Signalkonditionierer 2 elektrische Signale 101, 102, 103, 104, 105, 106, 107 oder 110 zur Verfügung gestellt. Der Signalkonditionierer 2 führt eine Signalvorverarbeitung durch, in dessen Verlauf der Signalanteil, der Informationen zur Rotorposition beziehungsweise Information zum Abstand zwischen einem Magnetfeldsensor und dem Rotor enthält, gegenüber anderen Signalanteilen relativ verstärkt wird. Dies kann beispielsweise durch frequenzselektive Filterung erfolgen, in dem nur die relevanten Informationen im Signal belassen werden oder auch durch Linearkombination mehrerer Messsignale. Das Ausgangssignal 120 des Signalkonditionierers 2 wird optional mit dem Analog-Digital-Umsetzer 3 in ein digitales Signal umgesetzt. Prinzipiell ist es möglich, dass der Analog-Digital-Umsetzer 3 in den Signalkonditionierer 2 integriert wird, so dass optional weitere Signalkonditionierungsverfahren auch nach dem Analog-Digital-Umsetzer 3 eingefügt werden können. Das Ausgangssignal 130 des Analog-Digital-Umsetzers 3 dient als Eingangssignal für den Demodulator 4, der ausgebildet ist, eine Amplitudendemodulation durchzuführen. Je nach dem, ob der optionale Analog-Digital-Umsetzer 3 verwendet wird, wird die Demodulation analog oder digital ausgeführt. Optional besteht darüber hinaus die Möglichkeit, dass, mit Hilfe der Vorrichtung 7 zur Bereitstellung der Rotordrehfrequenz 171 und des optionalen Rotordrehwinkels 172, die Rotordrehfrequenz 171 und/oder der Rotordrehwinkel 172 bei der Amplitudendemodulation verwendet wird. Aus den demodulierten Signalen 140 wird in der Vorrichtung 5 mindestens eine Komponente, vorzugsweise mehrere Komponenten der Rotorposition, insbesondere zumindest diejenige Komponente der Rotorposition, welche geregelt werden kann, bestimmt. Deren Ausgangssignal 150 wird in einer optionalen Steuereinheit 6 zur Erzeugung von Steuersignalen 160 verwendet. Optional können die gemessenen und/oder berechneten Signale und Daten in einer Datensammeleinheit 8 gespeichert werden.

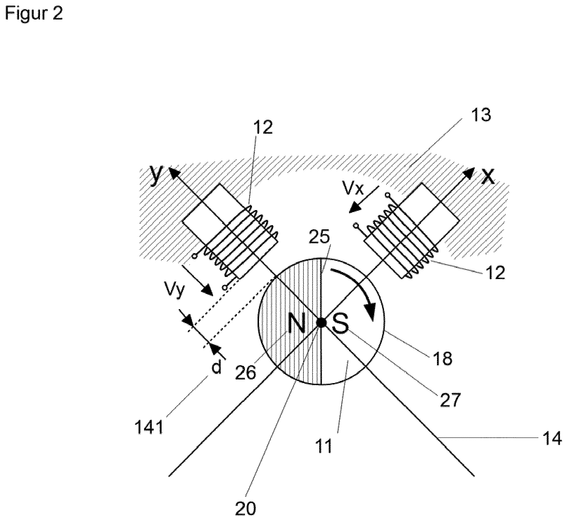

In Figur 2 wird eine Anordnung dargestellt, die den Rotor 11, Magnetfeldsensoren 12 in einem kartesischen Koordinatensystem 14 umfasst. Prinzipiell ist das Koordinatensystem 14 feststehend mit Bezug zum Stator 13 definiert, und zwar in einer Ebene senkrecht zur bezüglich des Stators feststehenden Achse 20. Die Rotordrehachsen-Sollposition wird in der Regel in den Koordinatenursprung dieses Koordinatensystems 14 gelegt. Die Magnetfeldsensoren 12 sind in diesem Beispiel jeweils in den Achsen des Koordinatensystems 14 so angeordnet, dass sie von einem rotorgenerierten Magnetfeld durchdrungen werden können. Der Rotor 11 enthält zumindest in dem axialen Abschnitt der Rotationsmaschine, in dem sich auch die Magnetfeldsensoren 12 befinden, Magneten 25, die in diesem Beispiel als Permamentmagneten, mit Nordpol 26 und Südpol 27, ausgeführt sind, aber auch Elektromagneten sein können. Über den Umfang 18 des Rotors 11 sind dementsprechend eine gerade Anzahl von magnetischen Polen verteilt, so dass bei Rotation des Rotors die Magnetfeldsensoren 12 einem periodisch wechselnden Magnetfeld ausgesetzt sind. Die Periodenfrequenz dieses wechselnden Magnetfeldes entspricht dem Produkt aus Rotordrehfrequenz 171 und der Polpaarzahl des Rotors. In diesem Beispiel sind die Magnetfeldsensoren 12 als Spulen ausgeführt, so dass in den Spulen 12 aufgrund des wechselnden magnetischen Flusses jeweils eine Spannung Vx bzw. Vy induziert wird, die proportional zur Änderung des magnetischen Flusses ist. Die induzierte Spannung Vx oder Vy hängt damit sowohl von der Rotordrehfrequenz 171 als auch vom Abstand 141 des Rotors 11 von der jeweiligen Spule 12 ab, der in diesem Beispiel jeweils eine radiale Position des Rotors 11 bezüglich der feststehenden Achse 20 darstellt.

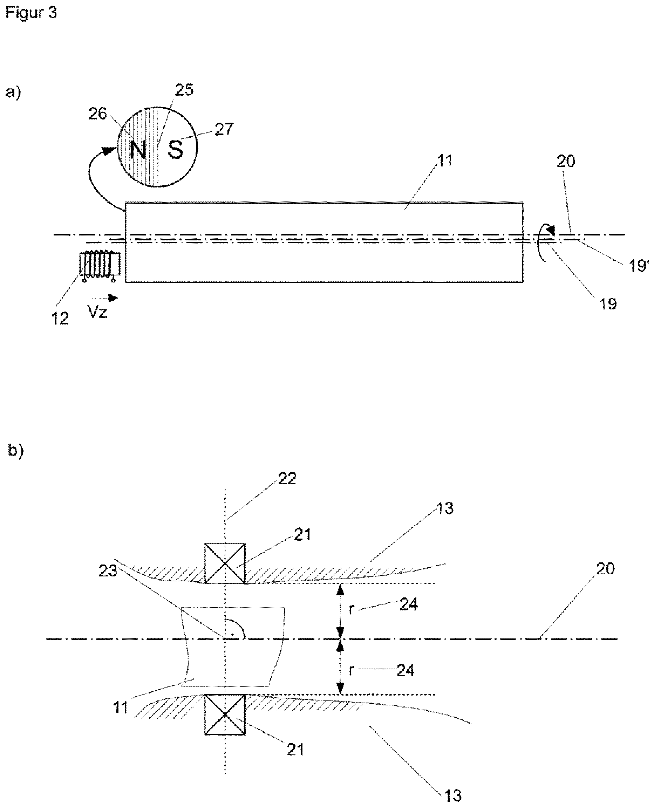

Figur 3a zeigt eine Anordnung mit Rotor 11 und Magnetfeldsensor 12. Der Rotor 11 enthält zumindest an einer Stirnseite einen Magneten 25 mit mindestens einem Nordpol 26 und einem Südpol 27, so dass der Magnetfeldsensor 12, der sich in einer axialen Richtung beabstandet vom Rotor 11 befindet, vom bei Rotation wechselnden Magnetfeld dieses Magneten 25 durchdrungen werden kann. Der Magnetfeldsensor 12 ist in dieser Abbildung als Spule 12 ausgeführt. Gemäß dem Induktionsgesetz wird in der Spule 12 eine Spannung induziert, die proportional zur Änderung des magnetischen Flusses ist. Die induzierte Spannung hängt damit sowohl von der Rotordrehfrequenz 171 als auch vom Abstand 140 des Rotors 11 von der Spule 12 ab, der eine axiale Position des Rotors bezüglich der feststehenden Position der Spule 12 angibt. Figur 3a zeigt ferner die bezüglich des Stators 13 feststehend definierte Achse 20. Die Rotorachse 19' befindet sich in der geometrischen Mitte des Rotorquerschnitts. Die Rotationsachse 19 kann, insbesondere bei Rotation bedingt durch externe Kräfte oder Unwuchten, von der Rotorachse 19' und der bezüglich des Stators feststehend definierten Achse 20 abweichen.

Figur 3b zeigt einen Ausschnitt des Rotors 11 im Bereich eines möglichen Lagers 21. Bei dem Lager kann es sich beispielsweise um ein Wälzlager, Gleitlager oder auch ein Magnetlager handeln. Die Position der bezüglich des Stators 13 feststehenden Achse 20 wird beispielsweise definiert durch den rechten Winkel zwischen der Lagerebene 22 und der Achse 20 sowie durch den Durchtrittspunkt 23 der Achse 20 durch die Lagerebene 22, die sich in der Lagermitte befindet, wobei die Lagermitte durch gleiche Beabstandung, r, 24 zu radial gegenüberliegenden Elementen des Lagers 21 definiert wird.

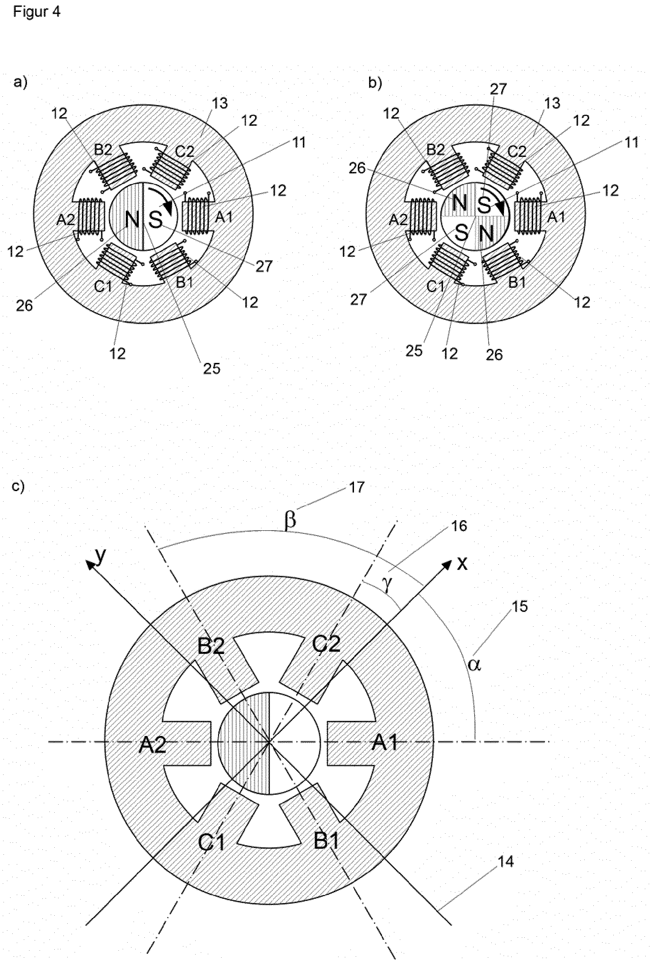

Figur 4a zeigt eine axiale Sicht auf eine Anordnung aus Rotor 11 und Stator 13, die typisch ist für eine Elektromotorkonfiguration. Dargestellt sind die Magnetfeldsensoren 12, die in einer Motoranwendung gleichzeitig als Antriebsspulen dienen können, die ausgebildet sind, ein oder mehrere Magnetfelder für die Erzeugung von Drehmoment zu erzeugen. Die Spulen sind als gegenüberliegende Paare A1-A2, B1-B2 und C1-C3 gekennzeichnet. In einer Motoranwendung können diese Paare jeweils elektrisch in Reihe geschaltet werden. Der Rotor 11 enthält eine Anordnung aus Magneten 25 mit einem Polpaar, das aus Nordpol 26 und Südpol 27 besteht.

Figur 4b zeigt analog zur Figur 4a eine axiale Sicht auf eine Anordnung aus Rotor 11 und Stator 13, die typisch ist für eine Elektromotorkonfiguration. Im Unterschied zur Figur 4a enthält der Rotor 11 eine Anordnung aus Magneten mit zwei Polpaaren, d.h. zwei Nordpolen 26 und zwei Südpolen 27. Prinzipiell können auch mehr als zwei Rotorpolpaare, bestehend aus jeweils einem Nordpol 26 und einem Südpol 27, oder mehr als auch weniger als die abgebildeten drei Spulenpaare, verwendet werden.

Figur 4c zeigt eine axiale Sicht auf eine Anordnung aus Rotor 11 und Stator 13 sowie das bereits definierte Koordinatensystem 14 mit seinen zwei orthogonal aufeinanderstehenden Achsen. Darüber hinaus sind die durch die Spulenpaare A1-A2, B1-B2 und C1-C2 definierten Achsen eingetragen, die durch die Drehwinkel $\alpha$ 15, $\beta$ 17 und $\gamma$ 16 von der x-Achse des Koordinatensystems 14 abweichen. Diese Winkel 15, 16 und 17 können genutzt werden, um die bezüglich der Sensoren 12 gemessenen Abstände 141 in Koordinaten des Koordinatensystems 14 umzurechnen.

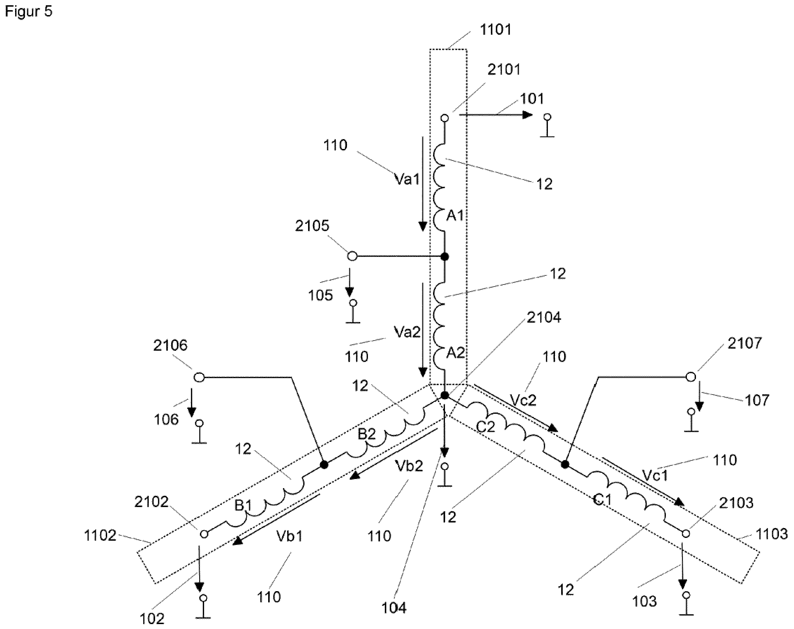

Figur 5 zeigt ein Beispiel die Verschaltung von Elektromotorspulen und deren Nutzung für die Messung von Magnetfeldern, d.h. als Magnetfeldsensoren 12. Dargestellt ist eine Sternschaltung, wobei der Motor für den motorischen Betrieb elektrisch an den Phasen-Anschlüssen 2101, 2102 und 2103 und dem Sternpunkt-Anschluss 2104 angeschlossen wird. Jeder Phasen-Anschluss 2101, 2102 und 2103 ist der Anschluss für eine elektrische Phase 1101, 1102 und 1103 des Motors, die jeweils aus einem Spulenpaar A1-A2, B1-B2 und C1-C2 besteht. Die Phasen 1101, 1102 und 1103 sind im Sternpunkt-Anschluss 2104 elektrisch miteinander verbunden. Darüber hinaus werden die Mittelabgriff-Anschlüsse 2105, 2106 und 2107 definiert. Ferner werden die Spannungen 101, 102, 103, 104, 105, 106 und 107 beispielsweise jeweils gegen Masse gemessen. Zusätzlich zu den über die Phasenanschlüsse 1101, 1102 und 1103 durch eine Motorsteuerung eingeprägten Spannungen oder Ströme werden bei Rotation des Rotors 11 Spannungen in den Spulen 12 induziert, die jeweils über den Spulen 12 A1, A2, Bl, B2 sowie C1 und C2 abfallen. Diese Darstellung zeigt somit ein Beispiel für eine elektrische Verschaltung der Magnetfeldsensoren 12, wenn sie als Motorspulen ausgeführt sind. Die Motorspulen sind damit ein Teil der Messvorrichtung 1. Die Spannungen 101, 102, 103, 104, 105, 106, 107 wie auch die jeweils insgesamt über den Spulen 12 abfallenden Spannungen, 110, Val, Va2, Vbl, Vb2, Vc1 und Vc2 können durch beispielsweise elektrische Verbinder und Anschlüsse und optional Messverstärker, Pegelumsetzer oder Impedanzwandler der nachfolgenden Verarbeitung zugänglich gemacht werden.

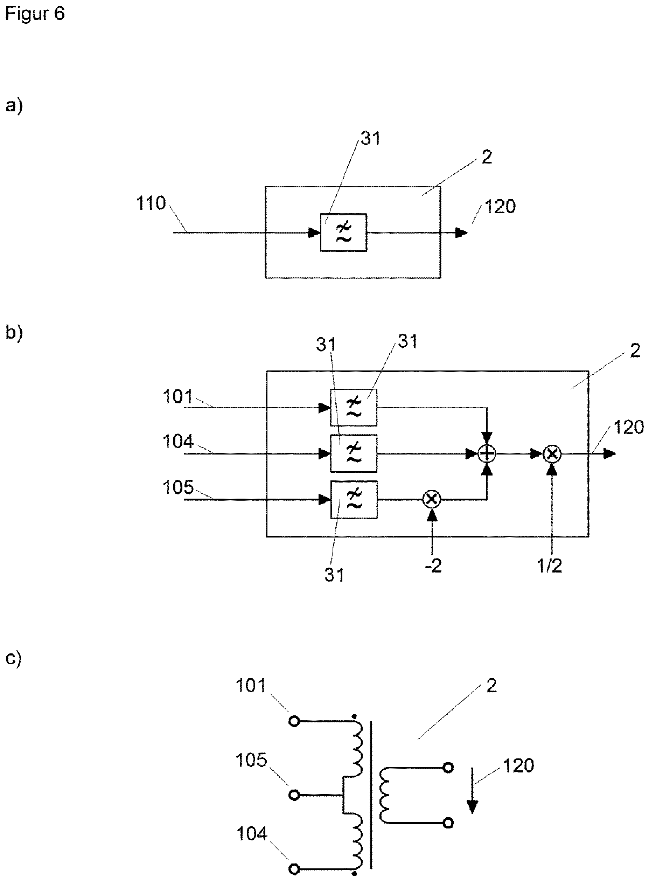

Die Signalkonditionierung 2, wird in den Teilen von Figur 6 gezeigt.

Figur 6a zeigt dabei einen Signalkonditionierer 2, der eine Spulenspannung 110 mit einem Filter 31 filtert, das als Tiefpassfilter ausgebildet ist. Durch die Tiefpassfilterung werden höherfrequente, für die Rotorpositionsbestimmung irrelevante Signalanteile aus dem Spulenspannungssignal 110 entfernt. Beispielsweise kann es sich bei den irrelevanten Signalanteilen um die pulsweitenmodulierten (PWM) Signale der Motorsteuerung handeln, die hochfrequente Schaltfrequenzen von beispielsweise 4 kHz oder 8 kHz aufweisen kann und deren Oberwellenanteile auf bis in den MHz-Bereich reichen können. Ein Tiefpassfilter, das ausgebildet ist, die pulsweitenmodulierten Signalanteile der Motorsteuerung zu unterdrücken, kann beispielsweise eine Sperrgrenzfrequenz von 3,9 kHz aufweisen, wobei die Sperrgrenzfrequenz die Frequenz ist, oberhalb welcher Signalkomponenten im Signal unterdrückt werden. Die Durchlassgrenzfrequenz eines solchen Tiefpassfilters kann beispielsweise so festgelegt werden, dass die für die Rotorpositionsbestimmung relevanten Signalkomponenten das Filter ungedämpft oder mit wenig Dämpfung passieren können. Die minimale Durchlassgrenzfrequenz ergibt sich in einem solchen Beispiel aus der Summe von Trägerschwingfrequenz 176 und maximaler Frequenz des Modulationssignals 179. Alternativ kann hier auch ein anderes Filter 31 genutzt werden, beispielsweise ein Bandpassfilter, wobei das Bandpassfilter beispielsweise als obere Durchlassgrenzfrequenz die Durchlassgrenzfrequenz des beschriebenen Tiefpassfilters und als obere Sperrgrenzfrequenz die Sperrgrenzfrequenz des beschriebenen Tiefpassfilters besitzen kann. Die untere Durchlassgrenzfrequenz des Bandpasses ergibt sich in einem solchen Beispiel aus der Trägerschwingfrequenz 176 abzüglich der maximalen Frequenz des Modulationssignals 179. Die untere Sperrfrequenz muss kleiner sein als die untere Durchlassgrenzfrequenz, kann aber beispielsweise ansonsten frei gewählt werden.

Figur 6b zeigt die Signalkonditionierung 2 durch Kombination mehrerer Messsignale. Kombiniert werden beispielhaft die Spannung der Phase A 101, die Spannung im Sternpunkt 104 sowie die Spannung am Mittelabgriff A 105. Alle drei Spannungen 101, 104, 105 werden beispielsweise gegen Masse gemessen. Zunächst werden alle drei Spannungssignale 101, 104 und 105 mit einem Filter 31, das als Tiefpassfilter ausgebildet ist, zur Entfernung der PWM-Signalanteile gefiltert, bevor sie anschließend gewichtet aufaddiert werden. Mit dieser Schaltung können beispielhaft Motorsignalanteile aus dem Spannungssignal entfernt werden, da mit dieser Schaltung die Differenz der in Figur 5 eingeführten Spannungen Va1 - Va2 ermittelt wird. Beide Spannungen enthalten bei angenommener Gleichheit der Spulen A1 und A2 eine auf die Motoransteuerung zurückzuführende Spannung Vm sowie die auf das modulierte Rotorpositionssignal zurückzuführende Spannung Vp, die jedoch aufgrund der gegenüberliegenden Anordnung der Spulen A1 und A2 unterschiedliche Vorzeichen besitzen. Die in Figur 6b gezeigte Schaltung berechnet somit Spannung

$$(Va1-Va2)\ /2 = ((Vm+Vp) - (Vm-Vp))\ /2 = 2Vp\ /2 = Vp.$$

Im Falle eines zweipoligen Läufers hält das Ergebnis der Gleichung ebenfalls.

Dieses Beispiel zeigt, dass die Kombination mehrerer Spulenspannungen 110 in bestimmten Ausführungsformen vorteilhaft zur Reduzierung der nicht für die Rotorpositionsbestimmung relevanten Signalanteile genutzt werden kann. Im konditionierten Signal 120 sind die der Motorsteuerung zugeordneten Signalkomponenten unterdrückt.

Figur 6c zeigt eine praktische Umsetzungsmöglichkeit des in Figur 6b gezeigten Signalkonditionierers 2 mit einem Transformator. Eine Alternative zu einer Transformatorschaltung bieten aus der Literatur bekannte aktive Rechenschaltungen mit Operationsverstärkern.

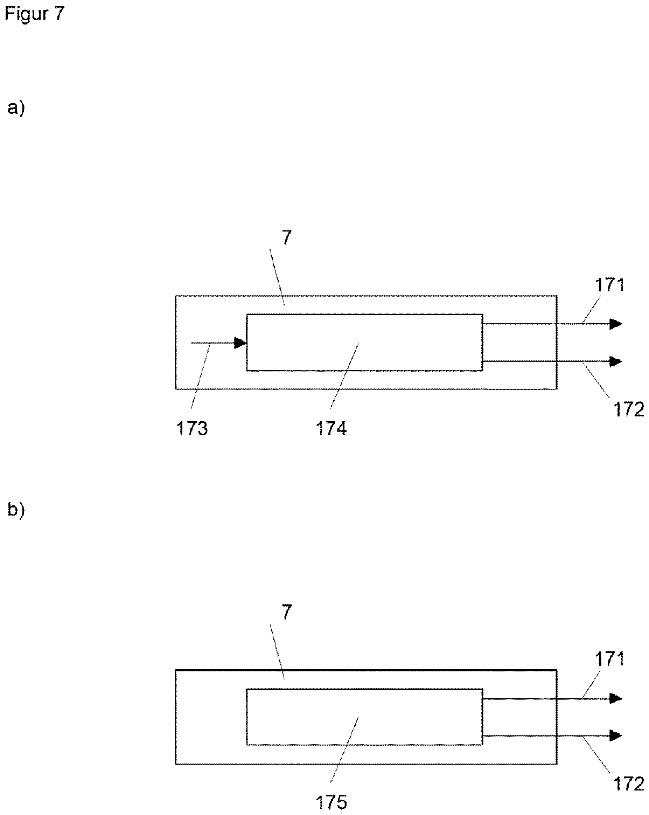

Figur 7a zeigt eine mögliche Implementierung einer Vorrichtung 7 zur Bereitstellung der Rotordrehfrequenz 171 und optional des Rotordrehwinkels 172. Als eine wesentliche Komponente der Vorrichtung 7 wird beispielhaft eine Zählerbaugruppe 174 gezeigt, die beispielsweise einen internen Zähler in einem gleichmäßigen Takt hochzählt. An einem Eingang erhält diese Zählerbaugruppe 174 ein Signal, beispielsweise einen Spannungsimpuls, das von einem Keyphasor 173 generiert wird. Beispielsweise kann dieser vom Keyphasor 173 generierte Spannungsimpuls durch weitere Baugruppen in einen Spannungsimpuls umgesetzt werden, der beispielsweise eine einheitliche vorbestimmte Spannung, beispielsweise 5V aufweist und beispielsweise auch eine einheitliche Länge, beispielsweise 50µs. Dieser Spannungsimpuls wird vom Keyphasor 173 immer dann generiert, wenn während einer Umdrehung des Rotors 11 eine Keyphasor-Nut den Keyphasor 173 passiert. Es werden deshalb so viele Spannungsimpulse pro Rotorumdrehung generiert, wie Keyphasor-Nuten auf dem Rotor angeordnet sind. Beispielhaft wird die weitere Verarbeitung des Spannungsimpulses für den Fall erläutert, dass genau eine Keyphasor-Nut auf dem Rotor 11 angeordnet ist. In der Zählerbaugruppe 174 wird der interne Zähler beispielsweise bei einer steigenden Signalflanke des Spannungsimpulses auf Null zurückgesetzt und der Zählvorgang, der eine getaktete Inkrementierung des Zählers beinhaltet, gestartet. Die Taktfrequenz wird dabei so festgelegt, dass pro Rotorumdrehung, auch bei Maximaldrehzahl, eine Vielzahl von Inkrementierungen durchgeführt wird. Bei Eintreffen derselben Flanke des nachfolgenden Spannungsimpulses wird der aktuell anstehende Zählerwert gespeichert, der Zähler auf Null zurückgesetzt und der Zählvorgang erneut gestartet. Die Rotordrehfrequenz 171 wird ermittelt, in dem die Taktfrequenz durch den gespeicherten Zählerwert geteilt wird. Für die Schätzung des Rotordrehwinkels während der nachfolgenden Umdrehung wird beispielsweise der jeweils aktuelle Zählerwert durch den gespeicherten Zählerwert geteilt und mit 360° multipliziert. Rotordrehfrequenz 171 und Rotordrehwinkel 172 werden am Ausgang der Vorrichtung 7 zur Verfügung gestellt. Figur 7b zeigt eine alternative Vorrichtung 7 zur Bereitstellung der Rotordrehfrequenz 171 und optional des Rotordrehwinkels 172, die bei einigen Motoren implementiert werden kann. Die Rotordrehfrequenz 171 und der Rotordrehwinkel 172 liegen beispielsweise in diesem Fall im Motor ohnehin vor und werden durch die Motorsteuerung 175 zur Verfügung gestellt.

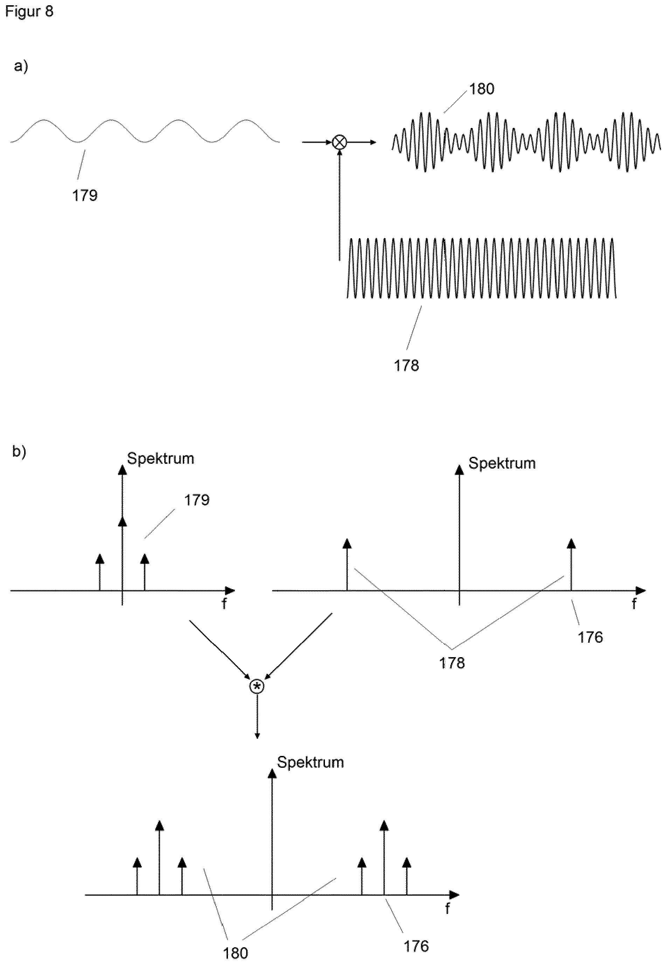

Figur 8a zeigt ein Signalbeispiel für die Erzeugung eines amplitudenmodulierten Signals 180 im Zeitbereich. Das amplitudenmodulierte Signal 180 entsteht aus der Multiplikation einer Trägerschwingung 178 mit einem Modulationssignal 179. Die Frequenz der Trägerschwingung 178 wird als Trägerschwingungsfrequenz 176 bezeichnet. Die Trägerschwingungsfrequenz 176 ist im amplitudenmodulierten Signal 180 sichtbar. Darüber hinaus schwankt die Amplitude des amplitudenmodulierten Signals 180 synchron zum Modulationssignal 179. In der offenbarten Rotationsmaschine entspricht das Modulationssignal 179 dem Abstand 141 zwischen einem Magnetfeldsensor 12 und dem Rotor 11. Das Trägerschwingungssignal entsteht in den Magnetfeldsensoren 12 durch die von den magnetischen Rotorpolen 26, 27 bedingten Änderungen des magnetischen Flusses in den Magnetfeldsensoren 12.

Figur 8b zeigt ein Signalbeispiel für die Erzeugung eines amplitudenmodulierten Signals 180 im Frequenzbereich. Das Spektrum, d.h. die Fouriertransformierte, des Modulationssignals 179 wird mit dem Spektrum, d.h. der Fouriertransformierten der Trägerschwingung 178 gefaltet. Im Ergebnis entsteht das Spektrum, d.h. die Fouriertransformierte des amplitudenmodulierten Signals 180. Das Spektrum des amplitudenmodulierten Signals 180 zeigt das Spektrum des Modulationssignals 179 jeweils um die Trägerschwingungsfrequenz 176 nach links, d.h. zu negativen Frequenzen, und um die Trägerschwingungsfrequenz 176 nach rechts, d.h. zu positiven Frequenzen verschoben.

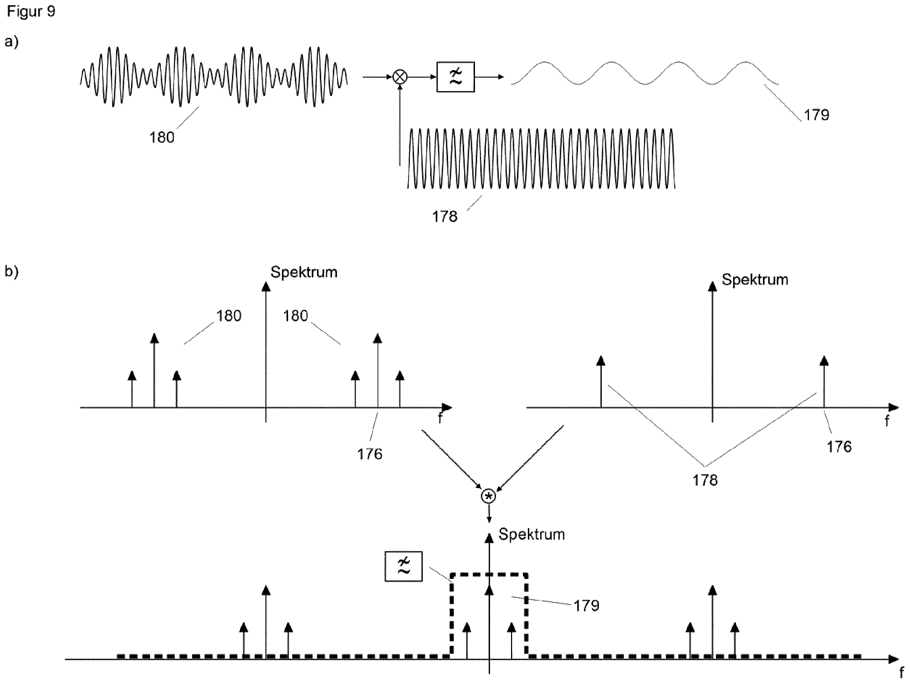

Figur 9a zeigt ein Signalbeispiel für die Amplitudendemodulation eines amplitudenmodulierten Signals 180 im Zeitbereich. Das amplitudenmodulierte Signal 180 wird mit der Trägerschwingung 178 multipliziert und im Anschluss mit einem Tiefpass gefiltert. Im Ergebnis entsteht das demodulierte Signal 179. Die Notwendigkeit des Tiefpasses wird in Figur 9b deutlich.

Figur 9b zeigt ein Signalbeispiel für die Amplitudendemodulation eines amplitudenmodulierten Signals 180 im Frequenzbereich. Das amplitudenmodulierte Signal 180, dessen Komponenten um die Trägerschwingungsfrequenz 176 angeordnet sind, wird mit dem Spektrum, d.h. der Fouriertransformierten, der Trägerschwingung 178 gefaltet. Im Ergebnis wird das Spektrum des Modulationssignals (siehe Figur 8b) in drei Bereichen sichtbar. Um die beiden nicht bei 0 Hz befindlichen Spektralbereiche zu unterdrücken und das Spektrum des Modulationssignals 179 zurückzugewinnen, wird das Signal mit einem Tiefpass gefiltert (gestrichelter Bereich).

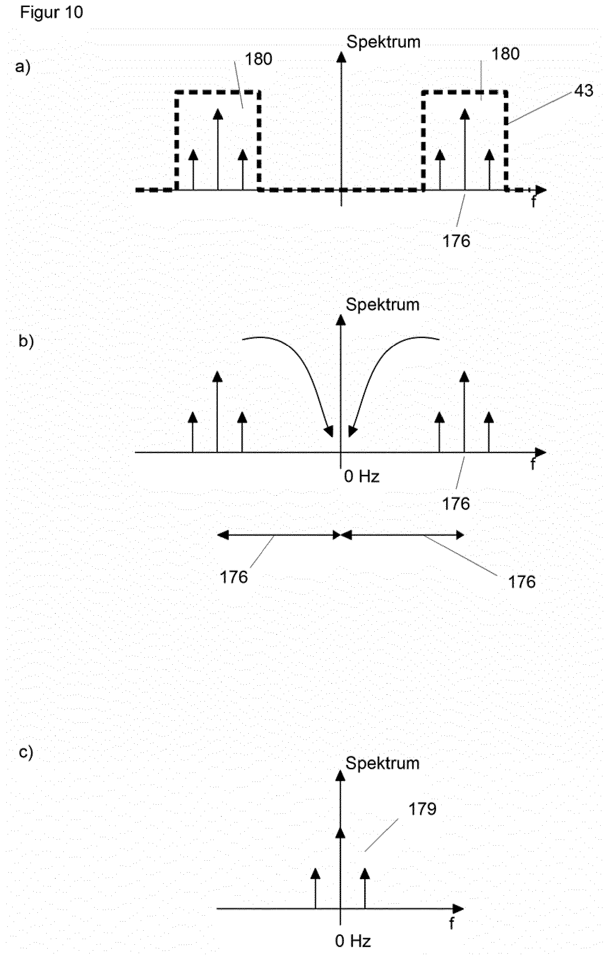

Figur 10 zeigt ein Beispiel für ein Demodulationsverfahren im Frequenzbereich.

Figur 10a zeigt das Spektrum des amplitudenmodulierten Signals 180. Es ist jeweils um die Trägerschwingungsfrequenz 176 angeordnet. Für eine Demodulation im Frequenzbereich werden alle Signalkomponenten, d.h. Fourierkoeffizienten, die nicht zum amplitudenmodulierten Signal gehören, durch die Vorrichtung zur Maskierung 43 maskiert, d.h. zu Null gesetzt.

Die eigentliche Demodulation wird in Figur 10b gezeigt. Die nach der Maskierung verbliebenen Signalanteile werden von der ursprünglichen Position zur Frequenz 0Hz verschoben, d.h. kopiert und am Ursprungsort gelöscht. Die Verschiebung erfolgt um einen Frequenzbetrag, der der Trägerschwingungsfrequenz 176 entspricht. Nach der Verschiebung an gleicher Stelle befindliche Koeffizienten werden addiert.

Figur 10c zeigt das durch diese Vorgehensweise entstehende Spektrum. Es entspricht dem Spektrum des Modulationssignals 179.

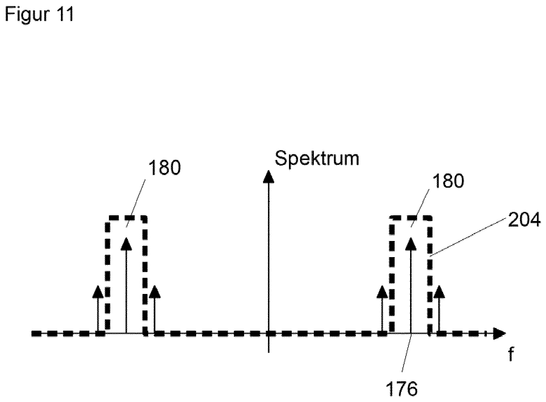

Figur 11 zeigt ein Beispiel für softwarebasierte Trägersynthese 201 durch Maskierung im Frequenzbereich. Dazu wird das amplitudenmodulierte Signal 180 im Frequenzbereich so maskiert, dass alle Signalanteile, die nicht zur Trägerschwingung 178 gehören, also Frequenzkomponenten besitzen, die nicht die Trägerschwingungsfrequenz 176 aufweisen, durch die Vorrichtung zur Peakerkennung und Maskierung 204 zu Null gesetzt werden. Das auf diese Weise maskierte Signal wird im Rahmen der softwarebasierten Trägersynthese 201 in den Zeitbereich transformiert. Falls die Trägerschwingungsfrequenz 176 nicht bekannt ist, kann sie beispielsweise durch eine Peakerkennung durch die Vorrichtung 204 geschätzt werden.

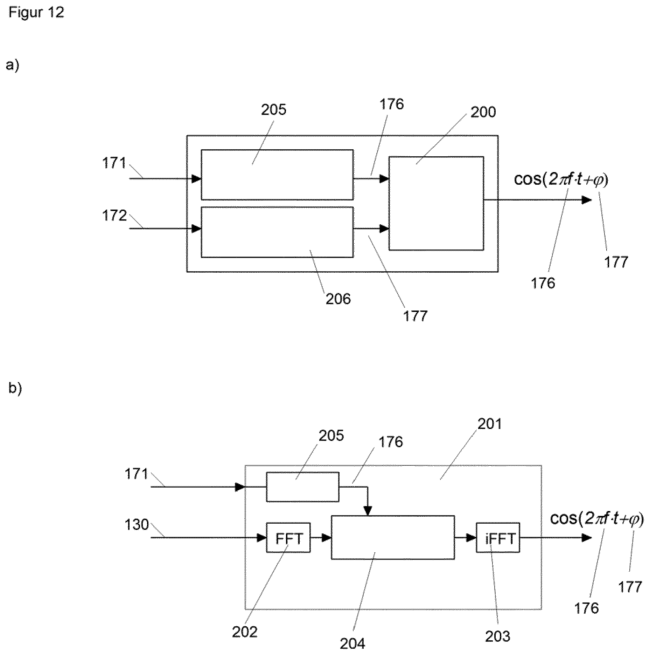

Figur 12a zeigt eine Möglichkeit, eine Trägerschwingung mit einer elektronischen Baugruppe zur Trägersynthese 200 zu erzeugen. Bei einer solchen Baugruppe 200 kann es sich beispielsweise um eine elektronische Oszillatorschaltung handeln, bei der eine Trägerschwingung 178 unter Nutzung der Vorgaben für die Frequenz und Phasenlage, die aus der Rotordrehfrequenz 171 und dem Rotordrehwinkel 172 erzeugt wird. Alternativ dazu kann die Trägersynthese 200 mit Hilfe Mikrocontrollers oder Computers erfolgen, indem ein in einem Speicher abgelegtes Signal abgerufen und mit einem Digital-Analog-Umsetzer in ein elektrisches Spannungssignal umgesetzt wird. In bestimmten Ausführungsformen besteht der Vorteil dieser Methode in einer größeren Flexibilität und einfacheren Konfigurierbarkeit. Die von der Trägersynthese 200 zu erzeugende Trägerschwingungsfrequenz 176 ergibt sich dabei aus dem Produkt von Rotordrehfrequenz und Polpaarzahl des Rotors. Sie wird in der Baugruppe 205 zur Berechnung der Trägerschwingungsfrequenz 176 berechnet. Die zu erzeugende Phasenlage 177 wir in der Baugruppe 206 zur Berechnung der Phasenlage 177 berechnet. Er muss für jedes Magnetfeldsensorsignal beziehungsweise zu demodulierende Signal individuell angepasst werden. Er hängt ab von den Positionen des Magnetfeldsensors und des statorfesten Referenzpunktes für den Rotordrehwinkel, von der aktuellen Position des rotorfesten Referenzpunktes für den Rotordrehwinkel sowie von der Polpaarzahl des Rotors.

Figur 12b zeigt ein Beispiel für eine softwarebasierte Trägersynthese 201 unter Nutzung der Diskreten Fouriertransformation. Dazu wird ein in den Digitalbereich umgesetztes Signal 130 beispielsweise durch die FFT-Transformationseinheit 202, die ausgebildet ist, eine schnelle Fouriertransformation (FFT) auszuführen, in den Frequenzbereich transformiert. Das transformierte Signal wird an eine Einheit für Peakerkennung und Maskierung 204, die ausgebildet ist Peaks in der Fouriertransformierten zu erkennen und Frequenzbänder oder Einzelfrequenzen zu maskieren, d.h., zu Null zu setzen, übergeben. Diese Einheit 204, maskiert alle Bereiche der Fouriertransformierten, die nicht in einem vordefinierten Bereich um die Trägerschwingungsfrequenz 176 liegen. Die Trägerschwingungsfrequenz 176 ergibt sich aus dem Produkt aus Rotordrehfrequenz 171 und der Polpaarzahl des Rotors. Das maskierte Signal wird in der iFFT-Einheit, die ausgebildet ist, eine inverse Fouriertransformation (iFFT) durchzuführen, in den Zeitbereich transformiert. Im Ergebnis liegt ein Trägerschwingungssignal mit der für die Demodulation geeigneten Trägerschwingungsfrequenz 176 und Phasenlage 177 vor.

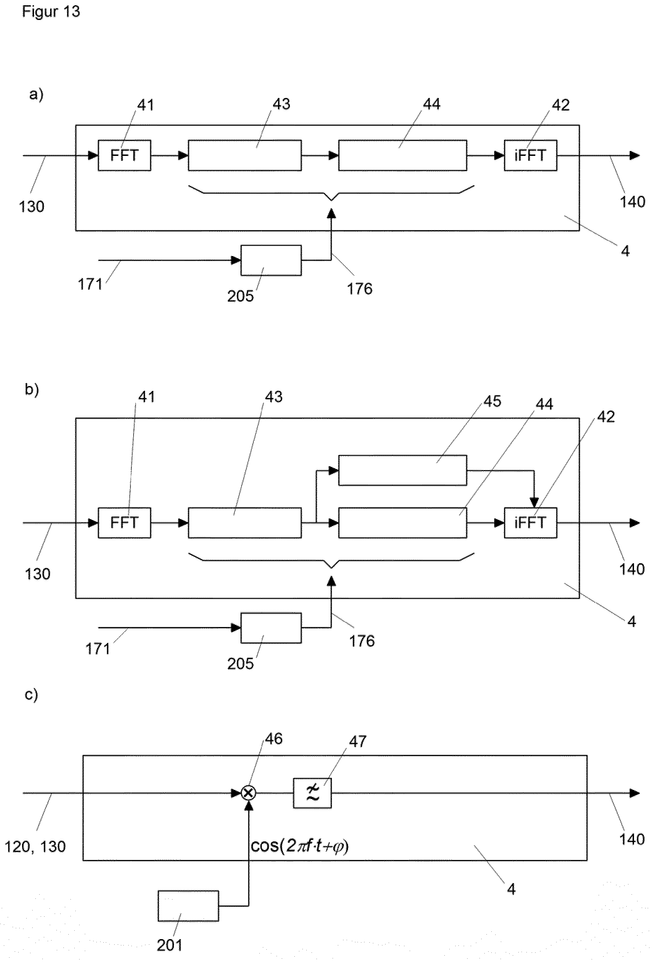

Figur 13 zeigt verschiedene Beispiele für den Demodulator in Zeit- und Frequenzbereich.

In Figur 13a ist ein Beispiel für den Demodulator 4 im Frequenzbereich mit Nutzung der Rotordrehfrequenz 171 und ohne Rotordrehwinkel 172. Dabei wird das digital vorliegende Signal 130 mit Hilfe der schnellen Fouriertransformation (FFT) 41 in den Frequenzbereich transformiert. In der Fouriertransformierten wird eine Maskierung 43 durchgeführt, d.h., alle nicht in der Umgebung der Trägerschwingungsfrequenz 176 liegenden Frequenzkomponenten werden zu Null gesetzt. Die Trägerschwingungsfrequenz 176 wird von der Einheit 205 für die Berechnung der Trägerschwingungsfrequenz 176 zur Verfügung gestellt. Sie ergibt sich aus dem Produkt von Rotordrehfrequenz 171 und der Polpaarzahl des Rotors. Es ist darauf zu achten, dass die Maskierung 43 sowohl für zu positiven als auch für zu negativen Frequenzen korrespondierenden Frequenzstützstellen durchzuführen ist. Im Weiteren erfolgt eine Verschiebung 44 der verbleibenden Frequenzkomponenten um den Betrag der Trägerschwingungsfrequenz 176 in Richtung der Frequenz 0 Hz. Dabei werden verschobene Frequenzen zu bereits um 0 Hz vorhandenen Frequenzanteilen jeweils komplex aufaddiert. Das resultierende Signal wird mit Hilfe einer inversen schnellen Fouriertransformation in den Zeitbereich zurücktransformiert. Das resultierende Signal 140 enthält keine korrekte Phaseninformation und kann deshalb nicht zur Berechnung der Rotorposition genutzt werden. Allerdings kann seine Leistung dazu verwendet werden, das aktuelle Schwingungsniveau des Rotors abzuschätzen.

Zusätzlich zu den in Figur 13a gezeigten Verarbeitungsstufen wird im Beispiel von Figur 13b eine Phasenkorrektur durchgeführt. Die Phasenkorrektur jedes der in Richtung 0Hz verschobenen Fourierkoeffizienten wird dabei so ausgeführt, dass sich der Betrag der korrigierten Phase aus dem Betrag der Differenz zwischen der unkorrigierten Phase und dem Phasenwert der Trägerschwingungsfrequenz 176 ergibt.

Figur 13c zeigt ein Beispiel für die Demodulation mit Trägerschwingungsfrequenz 176 und Phasenlage 177 im Zeitbereich. Hierbei wird das digital vorliegende Signal 130 mit dem von der Baugruppe zur Trägersynthese 201 synthetisierten Kosinussignal, das die Trägerschwingungsfrequenz 176 und die geschätzte Phasenlage 177 aufweist, mit Hilfe eines Multiplizierers 46 und einer anschließenden Tiefpassfilterung demoduliert. Der Tiefpass kann sowohl im Zeitbereich als digitales Filter realisiert werden oder auch im Frequenzbereich durch Maskierung zu entfernender Frequenzkomponenten.

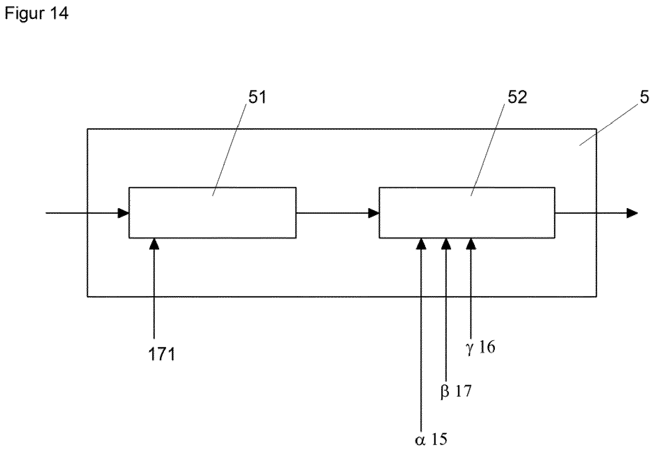

Figur 14 zeigt ein Beispiel für die Vorrichtung 5 zur Berechnung der Rotorposition. Die Vorrichtung 5 enthält als erste Verarbeitungsstufe eine drehzahlabhängige Skalierung 51, die unter Nutzung der Rotordrehfrequenz 171 und der Information zur Polpaarzahl des Rotors einen drehzahlabhängigen Korrekturfaktor berechnet. Dieser Korrektfaktor berücksichtigt, dass, wenn beispielsweise die Magnetfeldsensoren als Spulen ausgeführt sind, die induzierte Spannung proportional zur Änderung des magnetischen Flusses ist. Die Änderung des magnetischen Flusses ist jedoch direkt von der Rotordrehfrequenz 171 abhängig so dass dieser Effekt bei der Berechnung des Abstandes zwischen einem Magnetfeldsensor und dem Rotor eliminiert werden muss. Die drehzahlabhängige Skalierung 51 kann dabei beispielsweise als eine Tabelle oder ein Kennfeld ausgeführt und in einem Computer oder Mikroprozessor implementiert sein.

Die auf diese Weise korrigierten Abstandswerte werden anschließend für die Bestimmung der Rotorposition genutzt. Dazu wird für jeden Abstandswert eine Abweichung von einem vorgegebenen Sollwert berechnet, in dem von den korrigierten Abstandswerten jeweils der vordefinierte Sollwert subtrahiert wird. Über die bekannten Winkel $\alpha$ 15, $\beta$ 17 und $\gamma$ 16, die den Winkelversatz der durch die Magnetfeldsensoren 12 definierten Achsen gegenüber dem Koordinatensystem 14 angeben, kann die Position des Rotors von der jeweiligen Magnetfeldsensorachse auf die Koordinatenachsen des Koordinatensystems 14 über trigonometrische Beziehungen abgebildet werden. Die sich aus den verschiedenen Magnetfeldsensoren 12 ergebenden Koordinaten im Koordinatensystem 14 können beispielsweise durch Mittelwertbildung kombiniert werden.

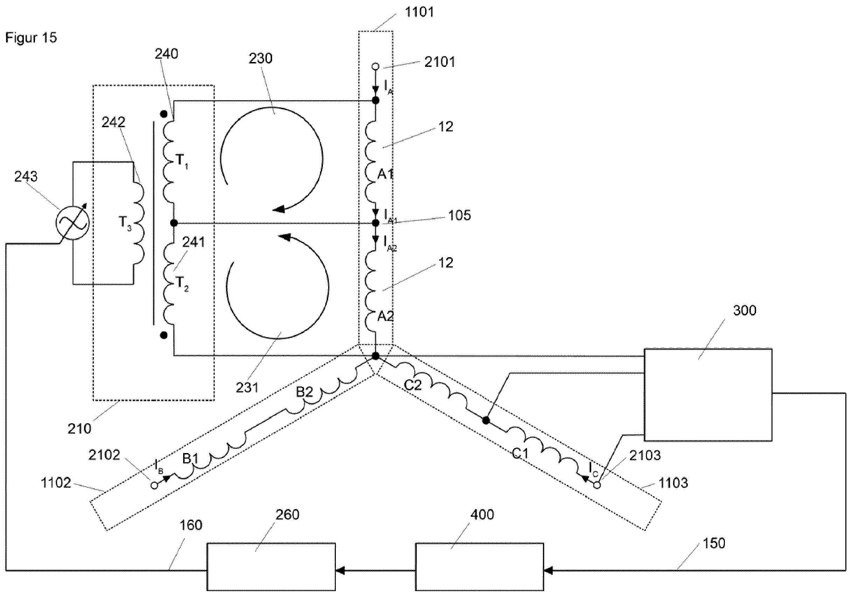

Figur 15 zeigt anhand eines Beispiels für einen Motor, wie das Rotorpositionssignal 150 für die Steuerung eines Aktuators genutzt werden kann. Interessant an diesem Beispiel ist, dass die Motorspulen, die ohnehin für die Erzeugung eines Antriebsmagnetfeldes benötigt werden, als Magnetfeldsensoren 12 und auch als Aktuatoren verwendbar sind. Figur 15 zeigt, dass beispielsweise die Rotorpositionserfassung im Phasenzweig C 1103 mit den über den Spulen C1 und C2 gemessenen elektrischen Spannungen erfolgt. Ein Regler 400, beispielsweise ein PID-Regler, der gegebenenfalls durch weitere Übertragungselemente ergänzt werden kann, berechnet Steuersignale, die dazu dienen, den Rotor in einer bestimmten Position zu halten oder aber auch, um das Schwingverhalten des Rotors zu dämpfen. Die Steuersignale werden mit dem Amplitudenmodulator 260 so transformiert, dass sie bezüglich des Rotordrehwinkels 172 eine Phasenlage besitzen, die zu einem stabilen Regelkreis führt. Das modulierte Signal wird mit Hilfe einer gesteuerten Stromquelle 243 in die Primärwicklung 242 eines Transformators eingeprägt, der zusammen mit den Sekundärwicklungen 240 und 241 die Aktuator-Einkoppeleinheit 210 bildet. Über die Sekundärwicklungen 240 und 241 wird ein Strom 230 in die Spule A1 eingeprägt und ein Strom 231 in die Spule A2 einprägt, wobei die Ströme in diesem Beispiel so orientiert sind, dass sie sich ihre Wirkung am Phasenanschluss A 2101 gegenseitig aufhebt und somit auch keinen Einfluss auf Spannungen und Ströme an den anderen Phasenanschlüsse 2102 und 2103 ausübt. Diese symmetrische Art und Weise, den Strom in die Phase A 1101 einzuprägen kann alternativ durch eine asymmetrische Art und Weise für die Einprägung des Stroms ersetzt werden, in dem man beispielsweise die Aktuator-Einkoppeleinheit 210 lediglich in eine Spule 12 A1 oder A2 einkoppeln lässt.

**Patentansprüche**

1. Rotationsmaschine mit

   - einem Stator (13) und einem drehbar gelagerten Rotor (11), der ausgebildet ist, sich bezüglich des Stators (13) zu bewegen, wobei in einem radialen Abstand von einer bezüglich des Stators (13) feststehenden Achse (20) ein oder mehrere Magnetfeldsensoren (12) bezüglich des Stators (13) feststehend angeordnet sind,
   - mindestens einer Messvorrichtung (1), die ausgebildet ist, Magnetfeldänderungen mit Hilfe der vorgenannten Magnetfeldsensoren (12) zu erfassen,
   - einem Rotor (11), der ausgebildet ist, mit einer oder mehreren konstanten magnetischen Quellspannungen und mit einem oder mehreren der Magnetfeldsensoren (12) jeweils ein oder mehrere elektrische Signale (101, 102, 103, 104, 105, 106, 107, 110) zu erzeugen, die Signalkomponenten aufweisen, die zur Rotordrehfrequenz (171) und zum jeweiligen Abstand zwischen Magnetfeldsensor (12) und Rotor (11) korrespondieren,
   **gekennzeichnet durch**
   - eine Demodulatoreinheit (4), die ausgebildet ist, bei von den Magnetfeldsensoren (12) erzeugten oder daraus abgeleiteten Signalen (101, 102, 103, 104, 105, 106, 107, 110, 120, 130), die Signalkomponenten aufweisen, die zur Rotordrehfrequenz (171) und zum jeweiligen Abstand zwischen Magnetfeldsensor (12) und Rotor (11) korrespondieren, eine Demodulation durchzuführen, so dass ein zum Abstand zwischen dem Rotor (11) und dem dem jeweiligen Signal zugeordneten Magnetfeldsensor (12) korrespondierendes Signal (140) erzeugt wird.

2. Rotationsmaschine nach Anspruch 1, **dadurch gekennzeichnet, dass** die Rotationsmaschine ein Motor ist.

3. Rotationsmaschine nach einem der vorhergehenden Ansprüche, **dadurch gekennzeichnet, dass** die Magnetfeldsensoren (12)

   - als Spulen ausgeführt sind und/oder
   - als Motorspulen ausgeführt sind und ausgebildet sind, Magnetfeldänderungen zu erfassen und ein für den Antrieb des Rotors geeignetes Magnetfeld zu erzeugen.

4. Rotationsmaschine nach einem der vorhergehenden Ansprüche, **gekennzeichnet durch**

   - eine Vorrichtung (7), die ausgebildet ist, die Rotordrehfrequenz (171) bereitzustellen,
   - Demodulatoreinheit (4), die ausgebildet ist, die Rotordrehfrequenz (171) bei der Demodulation zu verwenden.

5. Rotationsmaschine nach einem der vorhergehenden Ansprüche, **gekennzeichnet durch** eine erste Verarbeitungseinheit (2), die ausgebildet ist, ein oder mehrere elektrische Signale (101, 102, 103, 104, 105, 106, 107, 110) der vorgenannten Magnetfeldsensoren (12) zu einem oder mehreren Signalen (120) so zu überlagern und/oder zu filtern, dass der Signalanteil im jeweils resultierenden Signal, der eine Information zum Abstand (141) zwischen dem Rotor (11) und dem jeweiligen Magnetfeldsensor (12) enthält, jeweils im Verhältnis zu anderen Signalkomponenten verstärkt wird.

6. Rotationsmaschine nach einem der vorhergehenden Ansprüche, **gekennzeichnet durch**

   - eine zweite Verarbeitungseinheit (5), die dem Demodulator (4) nachgeschaltet ist und die ausgebildet ist, aus den demodulierten Signalen (140) ein oder mehrere Rotorpositionssignale (150) zu erzeugen und
- vorzugsweise eine Steuereinheit (6), die ausgebildet ist, aus den Rotorpositionssignalen (150) Steuersignale (160) zu generieren.

7. Rotationsmaschine nach einem der vorhergehenden Ansprüche, **gekennzeichnet durch** eine Datensammeleinheit (8), die ausgebildet ist, einen oder mehrere ermittelte Positionswerte des Rotors (150) zu speichern.

8. Verfahren unter Verwendung einer Anordnung nach einem der vorhergehenden Ansprüche, **dadurch gekennzeichnet, dass**

- ein oder mehrere elektrische Signale (101, 102, 103, 104, 105, 106, 107, 110) an den Magnetfeldsensoren (12) gemessen werden und
- ein oder mehrere an den Magnetfeldsensoren (12) gemessene oder daraus abgeleitete Signale (101, 102, 103, 104, 105, 106, 107, 110, 120, 130) demoduliert werden.

9. Verfahren nach Anspruch 8, **dadurch gekennzeichnet, dass** die Rotordrehfrequenz (171) zur Demodulation und vorzugsweise der Rotordrehwinkel (172) zur Demodulation genutzt wird.

10. Verfahren nach einem der Ansprüche 8 bis 9, **dadurch gekennzeichnet, dass** ein oder mehrere elektrische Signale (101, 102, 103, 104, 105, 106, 107, 110) der vorgenannten Magnetfeldsensoren (12) zu einem oder mehreren Signalen (120) so verarbeitet werden, dass der Signalanteil im jeweils resultierenden Signal, der eine Information zum Abstand (141) zwischen dem Rotor (11) und dem jeweiligen Magnetfeldsensor (12) enthält, jeweils im Verhältnis zu anderen Signalkomponenten verstärkt wird.

11. Verfahren nach einem der Ansprüche 8 bis 10, **dadurch gekennzeichnet, dass** aus den demodulierten Signalen (140) mindestens eine oder mehrere Komponenten einer Rotorposition (150) und/oder eine lineare Verschiebungsgeschwindigkeit und/oder eine lineare Beschleunigung der Rotorachse (19') ermittelt wird.

12. Verfahren nach Anspruch 11, **dadurch gekennzeichnet, dass** aus den Rotorpositionssignalen (150) Steuersignale (160) generiert werden.

13. Verfahren nach einem der Ansprüche 11 bis 12, **dadurch gekennzeichnet, dass** aus den Rotorpositionssignalen (150) eine einwirkende Kraft und/oder ein einwirkendes Drehmoment auf den Rotor ermittelt wird.

14. Verfahren nach einem der Ansprüche 11 bis 13, **dadurch gekennzeichnet, dass** ein oder mehrere ermittelte Rotorpositionswerte (150) in einer Datensammeleinheit (8) gespeichert werden.

15. Blutpumpensystem mit einer Rotationsmaschine nach einem der Ansprüche 1 bis 7.

Figur 1

EP 3 825 657 A1

Figur 2

Figur 3

a)

b)

Figur 4

a)

b)

c)

Figur 5

Figur 6

a)

b)

c)

Figur 7

a)

b)

Figur 8

a)

180

179

178

b)

Spektrum

179

f

Spektrum

178

176

f

⊛

Spektrum

180

176

f

Figur 9

Figur 10

Figur 11

Figur 12

a)

$$\cos(2\pi f \cdot t + \varphi)$$

b)

$$\cos(2\pi f \cdot t + \varphi)$$

Figur 13

a)

b)

c)

$$\cos(2\pi f \cdot t + \varphi)$$

Figur 14

Figur 15

**Europäisches Patentamt**
**European Patent Office**
**Office européen des brevets**

# EUROPÄISCHER RECHERCHENBERICHT

Nummer der Anmeldung

EP 19 21 1040

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 589 827 A1 (ETH ZUERICH [CH]) 8. Mai 2013 (2013-05-08) | 1-14 | INV. G01D5/14 |
| Y | * Absätze [0001] - [0003], [0010], [0011], [0045] - [0072] * | 15 | G01D5/12 G01D5/20 F16C32/04 |
| | ----- | | |
| X | DE 695 03 613 T2 (SOC D MECANIQUE MAGNETIQUE [FR]) 25. Februar 1999 (1999-02-25) | 1,5-12, 14 | |
| Y | * Absätze [0031] - [0064]; Abbildung 1 * | 15 | |
| | ----- | | |
| Y,D | US 8 226 373 B2 (YAEGASHI MITSUTOSHI [JP]; TERUMO CORP [JP]) 24. Juli 2012 (2012-07-24) * das ganze Dokument * | 15 | |
| | ----- | | |

RECHERCHIERTE SACHGEBIETE (IPC)

G01D
F16C

Der vorliegende Recherchenbericht wurde für alle Patentansprüche erstellt

| Recherchenort | Abschlußdatum der Recherche | Prüfer |
|---|---|---|
| München | 12. Mai 2020 | Stobbelaar, Mark |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 19 21 1040

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-05-2020

| Im Recherchenbericht angeführtes Patentdokument | Datum der Veröffentlichung | Mitglied(er) der Patentfamilie | Datum der Veröffentlichung |
|---|---|---|---|
| EP 2589827 A1 | 08-05-2013 | EP 2589827 A1 | 08-05-2013 |
| | | EP 2773879 A2 | 10-09-2014 |
| | | JP 6189310 B2 | 30-08-2017 |
| | | JP 2014532868 A | 08-12-2014 |
| | | KR 20140094555 A | 30-07-2014 |
| | | US 2014252899 A1 | 11-09-2014 |
| | | WO 2013063709 A2 | 10-05-2013 |
| DE 69503613 T2 | 25-02-1999 | DE 69503613 D1 | 27-08-1998 |
| | | DE 69503613 T2 | 25-02-1999 |
| | | EP 0749538 A1 | 27-12-1996 |
| | | FR 2716700 A1 | 01-09-1995 |
| | | JP 3382947 B2 | 04-03-2003 |
| | | JP H09510280 A | 14-10-1997 |
| | | US 5844339 A | 01-12-1998 |
| | | WO 9523297 A1 | 31-08-1995 |
| US 8226373 B2 | 24-07-2012 | JP 4787726 B2 | 05-10-2011 |
| | | JP 2008132131 A | 12-06-2008 |
| | | US 2008124231 A1 | 29-05-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9506475 B2
- US 8226373 B2